# EUROPÄISCHE PATENTSCHRIFT

(11) **EP 3 476 421 B1**
(45) Veröffentlichungstag und Bekanntmachung des Hinweises auf die Patenterteilung: **07.04.2021**
(21) Anmeldenummer: 18200453.1
(22) Anmeldetag: 15.10.2018
(51) Int. Cl.: A61M 11/02, A61M 11/06, A61M 39/12, B05B 7/24, B05B 15/63, B05B 15/65, B05B 9/01

(54) **APPLIKATOR MIT VEREINFACHTER REINIGUNG**
APPLICATOR WITH SIMPLIFIED CLEANING
APPLICATEUR À NETTOYAGE SIMPLIFIÉ

(30) Priorität: 24.10.2017 DE 202017005478 U
(43) Veröffentlichungstag der Anmeldung: 01.05.2019
(73) Patentinhaber: Kemper, Thomas, 22339 Hamburg (DE)
(72) Erfinder: Kemper, Thomas, 22339 Hamburg (DE)
(74) Vertreter: Misselhorn, Hein-Martin

(56) Entgegenhaltungen:
- DE-A1- 3 108 918
- FR-A1- 2 697 755
- US-A- 1 509 671
- US-A- 3 743 188
- US-A- 4 518 122
- US-A1- 2016 206 819
- US-B1- 9 016 671

## Beschreibung

Die Erfindung betrifft ein Applikatorsystem nach dem Oberbegriff des Anspruchs 1.

### TECHNISCHER HINTERGRUND

Marktübliche, extern gespeiste Applikatorsysteme und ihre Handstücke für manuelles Dosieren und Ausbringen von Flüssigkeiten und Gasen im medizinischen Bereich sind meist fest mit ihrem Schlauchsystem verbunden. Ein Lösen der Versorgungschläuche ist meist mit einem Werkzeug möglich. Es ist mit einem hohen Zeitaufwand verbunden. Aufgrund dessen sind die bekannten Handstücke zum manuellen Dosieren und Ausbringen von Flüssigkeiten und Gasen im medizinischen Bereich nur schlecht zu reinigen.

Aus den Druckschriften US 3,743,188, US 4,518,122 und US 1,509,671 sind Sprühpistolen zum Aufsprühen von Farbe und/oder Lack gezeigt. Die Sprühpistolen umfassen Versorgungsleitungen, mit welchen die Farbe zur Sprühpistole zugeführt wird. Die in diesen Druckschriften gezeigten Sprühpistolen eignen sich, insbesondere aus hygienischen Gründen, nicht für medizinische Zwecke.

Die Druckschrift US 2016/0206819 A1 zeigt eine Spritze mit einem Luer-Lock-Verschluss. Die Spritze ist über einen Tank befüllt. Ein extern gespeistes Applikatorsystem geht aus dieser Druckschrift nicht hervor.

Die Druckschrift US 9,016,671 B1 zeigt einen Aerosolgenerator zur Erzeugung von Aerosolen. Der Aerosolgenerator wird eingesetzt zu Forschungszwecken im Bereich der Inhalation von hochtoxischen Substanzen.

Die FR 2 697 755 A1 zeigt eine Vorrichtung zum Versprühen von antibiotischen oder antiseptischen Lösungen zum Reinigen und Behandeln von Wunden, wobei die Lösung in einem Medikamentenzerstäuber beinhaltet ist und über eine Gasströmung mitgerissen wird. Die Gasströmung wird über einen Gasschlauch zugeführt. Ein extern gespeistes Applikatorsystem geht aus dieser Druckschrift nicht hervor.

Die DE 31 08 918 A1 zeigt einen Medikamentenzerstäuber, der zum Spülen und Einsprühen von Körperhöhlen, insbesondere Knochenlagern, in denen Endprothesen mit Knochenzement fixiert werden sollen, verwendet wird.

Seit geraumer Zeit ist das Problem der multiresistenten Keime virulent, weshalb im medizinischen Bereich immer höhere Anforderungen an die Hygiene gestellt werden. Aufgrund dessen sollen auch Applikatoren regelmäßig gründlich und in ihre Einzelteile zerlegt gereinigt, desinfiziert und/oder autoklaviert werden. Dabei müssen die Versorgungschläuche abgekoppelt werden, denn sie lassen sich nicht sinnvoll und zuverlässig der regelmäßigen Reinigung oder Autoklavierung unterziehen.

### DAS DER ERFINDUNG ZUGRUNDE LIEGENDE PROBLEM

Dementsprechend liegt der Erfindung das Problem zugrunde, ein Applikatorsystem zu schaffen, bei dem sich die Versorgungschläuche schnell und zuverlässig vom Handstück trennen und auch wieder mit ihm verbinden lassen, vorzugsweise ohne dass mit steigender Anzahl von Trennungen und wieder Verbindungen die Gefahr einer Undichtigkeit signifikant ansteigt.

### DIE ERFINDUNGSGEMÄSSE LÖSUNG

Die erfindungsgemäße Lösung besteht in einem Applikatorsystem nach Maßgabe des Anspruchs 1.

Es wird ein Applikatorsystem mit einem Applikator vorgeschlagen, der ein Handstück umfasst sowie einen Dosator, einen Versorgungsschlauch, der den Applikator mit der zu applizierenden Substanz speist, und ein mit dem Schlauch fluiddicht verbundenes Kupplungsteil.

Dabei sind das Handstück und der Dosator lösbar, und vorzugsweise werkzeuglos mit bloßer Hand lösbar, miteinander gekoppelt. Meist erfolgt die Koppelung durch eine Gewindeverbindung, alternativ ist aber beispielsweise auch eine Koppelung durch Einschieben eines oder mehrerer Stifte - etwa senkrecht zur Längsachse - denkbar.

Das erfindungsgemäße Applikatorsystem zeichnet sich dadurch aus, dass der Dosator eine Kupplungsmuffe ausbildet, in die der Kupplungsnippel eines mit dem Versorgungsschlauch verbundenen Kupplungsteils fluiddicht eingeschoben ist, und dass der Kupplungsnippel durch das Kuppeln des Dosators mit dem Handstück in Längsachsenrichtung in der Kupplungsmuffe festgesetzt wird.

Alternativ wird, im Sinne eines weiteren Hauptanspruchs, auch beansprucht, dass der Dosator einen Kupplungsnippel ausbildet, der in eine Kupplungsmuffe eines mit dem Versorgungsschlauch verbundenen Kupplungsteils fluiddicht eingeschoben ist, wobei auch in diesem Fall der Kupplungsnippel durch das Kuppeln des Dosators mit dem Handstück in Längsachsenrichtung in der Kupplungsmuffe festgesetzt wird.

Unter einer Kupplungsmuffe im Sinne der Erfindung wird ein Rohrabschnitt verstanden, der in seinem Inneren einen Aufnahmeraum für einen Kupplungsnippel bildet, wobei die Innenmantelfläche des besagten Rohrabschnitts eine Dichtfläche bildet oder eine Dichtung trägt, vorzugsweise in Gestalt einer Schnurdichtung. Unter einem Kupplungsnippel im Sinn der Erfindung wird ein Rohrabschnitt zum Einschieben in eine Kupplungsmuffe verstanden, der an seiner Außenmantelfläche eine Dichtfläche bildet oder eine Dichtung trägt, vorzugsweise in Gestalt einer Schnurdichtung.

Dadurch wird eine Fluidkupplung geschaffen, deren Dichtigkeit auch durch häufig wiederholtes Trennen und wieder Fügen nicht beeinträchtigt wird.

Aufgrund der Tatsache, dass der Kupplungsnippel durch das Kuppeln des Dosators mit dem Handstück in Längsachsenrichtung in der Kupplungsmuffe festgesetzt wird, kann eine besonders einfache und schnelle Zerlegbarkeit und wieder Zusammenbaubarkeit erreicht werden. Besonders bevorzugt sind die Zerlegung und der Zusammenbau werkzeuglos möglich, mit bloßen Händen. Es genügt, den Dosator vom Handstück abzukuppeln, wodurch dann auch gleich der nächste Schritt des Abnehmens des Versorgungsschlauchs und seines Kupplungselements vom Handstück freigegeben wird.

Das führt im Zuge der meist in engen Abständen erforderlichen Reinigungsarbeiten immer wiederkehrend zu einer erheblichen Zeitersparnis. Vorzugsweise erübrigt es sich, am hygienisch sensiblen Einsatzort Werkzeug bereithalten zu müssen, mit dem die Zerlegung und der Zusammenbau bewerkstelligt werden und das entweder selbst regelmäßig sterilisiert werden muss oder einen hygienischen Risikofaktor darstellt.

### OPTIONALE AUSGESTALTUNGSMÖGLICHKEITEN DER ERFINDUNG

Vorzugsweise sind die Kupplungsmuffe und der Kupplungsnippel derart gestaltet, dass sie auch im betriebsbereiten Zustand des Applikators relativ zueinander rotieren können. Dabei wird das Kupplungsteil idealerweise so von dem Handstück gehalten, dass es auch im betriebsbereiten Zustand des Applikators relativ zum Handstück rotieren kann. Auf diese Art und Weise ist ein sehr freies Arbeiten mit dem Applikator möglich, das nicht durch Spannungen behindert wird, die sonst im Laufe der Zeit durch eine mehrfache Verdrehung des Versorgungsschlauchs auftreten.

Vorzugsweise ist vorgesehen, dass das Kupplungsteil in betriebsbereitem Zustand des Applikators derart in dem Handstück eingehängt ist, dass es formschlüssig gegen Herausziehen aus dem Handstück in Richtung der Längsachse L des Versorgungsschlauchs gesichert ist. Dadurch wird zwangsweise die erfindungsgemäße Fixierung zwischen Kupplungsnippel und Kupplungsmuffe erreicht, sobald das Handstück mit dem Dosator verbunden ist.

Bevorzugt ist eine Lösung, bei der das Kupplungsteil und das Handstück derart ausgestaltet sind, dass das Kupplungsteil mitsamt des daran befestigt bleibenden Schlauchs werkzeuglos von dem Handstück getrennt werden kann, sobald der Dosator von dem Handstück abgekuppelt ist. Auf diese Art und Weise können die zu reinigenden Teile allesamt sehr einfach von dem Versorgungsschlauch und dem dauerhaft daran befestigten Kupplungsstück getrennt werden, um sie zu Reinigungszwecken hiervon zu entfernen.

Vorzugsweise ist es so, dass das Kupplungsteil und das Handstück derart ausgestaltet sind, dass das Kupplungsteil nach dem Abziehen eines Halteorgans, das das Kupplungsteil in betriebsbereitem Zustand formschlüssig gegen Herausziehen aus dem Handstück sichert, zu der dem Dosator abgewandten Seite des Handstücks hin aus dem Handstück herausgezogen werden kann.

Eine bevorzugte Lösung sieht vor, dass das Halteorgan erst nach dem Abkuppeln des Dosators vom Handstück abgezogen werden kann, vorzugweise dadurch, dass das Anschlussstück, das Kupplungsteil und das Halteorgan derart gestaltet sind, dass das Kupplungsstück nach dem Abkuppeln des Dosators auf der Dosatorseite des Handstücks so weit aus diesem herausgeschoben werden kann, dass nun das in betriebsbereitem Zustand vollständig unzugänglich innerhalb des Handstücks untergebrachte Halteorgan zugänglich wird und abgezogen werden kann. Auf diese Art und Weise ist das Halteorgan im regulären Betrieb unzugänglich untergebracht, wodurch sichergestellt ist, dass das Halteorgan nicht unbeabsichtigt gelöst oder vollständig abgezogen wird und sich dann der Versorgungsschlauch unbeabsichtigt vom Applikator trennen kann.

Vorzugsweise weist der Dosator eine Kupplungsmuffe auf, die an ihrer Außenumfangsfläche ein Außengewinde trägt, mittels dessen der Dosator mit einem entsprechenden Innengewinde des Handstücks verschraubt werden kann. Dabei ist das Gewinde vorzugsweise so angeordnet, dass es bei betriebsbereit montiertem Applikator, in Richtung senkrecht zur Längsachse des angrenzenden Schlauchs gesehen, zwischen dem Innengewinde des Handstücks und dem in die Kupplungsmuffe eingeschobenen Kupplungsnippel liegt. Auf diese Art und Weise lässt sich eine besonders kompakte und handliche Gestaltung des Applikators erreichen.

Idealerweise weist der Dosator eine Kupplungsmuffe auf, deren freie Stirnfläche bei vollständig in das Handstück eingeschraubtem Dosator eine Anschlagfläche für das Halteorgan bildet. Auf diese Art und Weise wird das Kupplungsstück in vollständig montiertem Zustand sicher gehalten und die Montage kann trotzdem so erfolgen, dass erst die Verbindung zwischen dem Kupplungsnippel und der Kupplungsmuffe hergestellt wird und erst dann der Dosator und das Handstück miteinander gekoppelt werden.

Besonders bevorzugt ist, wenn der Dosator eine zweistufige Kupplungsmuffe ausbildet, mit einem ersten Muffenabschnitt mit einem großen Durchmesser und einem zweiten Muffenabschnitt mit einem kleinen Durchmesser. Dabei weist das Applikatorsystem unterschiedliche Kupplungsteile auf, von denen eines einen einstufigen Kupplungsnippel ausbildet und das andere einen zweistufigen Kupplungsnippel. Der Dosator ist so gestaltet, dass nach dem Einschieben eines einstufigen Kupplungsnippels in die Kupplungsmuffe Fluid in statischer Form am Dosator ansteht, das nur im Zuge der Applikation fließt, und dass nach dem Einschieben eines zweistufigen Kupplungsnippels das zu applizierende Fluid dynamisch am Dosator ansteht, indem es permanent durch den Dosator zirkuliert egal ob gerade appliziert wird oder nicht.

Weitere Vorteile, Wirkungsweisen und Ausgestaltungsmöglichkeiten ergeben sich anhand der nachfolgenden Beschreibung der Ausführungsbeispiele anhand der Figuren.

### FIGURENLISTE

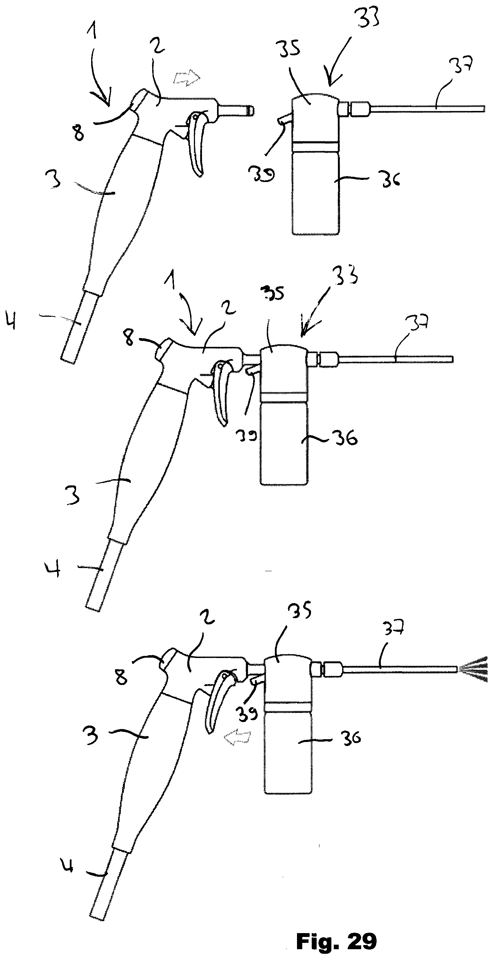

Die Figur 1 zeigt einen Applikator 1 des erfindungsgemäßen Applikatorsystems in Seitenansicht und in betriebsbereit montiertem Zustand.
Die Figur 2 zeigt eine explodierte Darstellung des Applikators gemäß Figur 1.
Die Figur 3 zeigt ein Ausführungsbeispiel eines Applikators 1 des erfindungsgemäßen Applikatorsystems im Mittellängsschnitt und bei unbetätigtem Abzugshebel, wobei dieser Applikator mit einem zweiflutigen Versorgungsschlauch in Verbindung steht.
Die Fig. 3a zeigt eine Ausschnittvergrößerung aus Fig. 3.
Die Figur 4a zeigt die Kombination aus einem zweiflutigen Versorgungsschlauch und dem daran angeschlossenen Kupplungselement, von der Seite her gesehen.
Die Figur 4b zeigt einen Mittellängsschnitt durch die Figur 4a. Die Figur 4c zeigt die Bestandteile der Figur 4a in explodiertem Zustand.
Die Figur 4d zeigt die Bestandteile der Figur 4b in explodiertem Zustand.
Die Figur 5 zeigt ein Ausführungsbeispiel eines Applikators 1 des erfindungsgemäßen Applikatorsystems im Mittellängsschnitt und bei unbetätigtem Abzugshebel, wobei dieser Applikator mit einem einflutigen Versorgungsschlauch in Verbindung steht.
Die Figur 6a zeigt die Kombination aus einem einflutigen Versorgungsschlauch und dem daran angeschlossenen Kupplungselement, von der Seite her gesehen.
Die Figur 6b zeigt einen Mittellängsschnitt durch die Figur 6a. Die Figur 6c zeigt die Bestandteile der Figur 6a in explodiertem Zustand.
Die Figur 6d zeigt die Bestandteile der Figur 6b in explodiertem Zustand.
Die Figur 7 zeigt den Applikator gemäß Figur 3 bei betätigtem Abzugshebel.
Die Figur 8 zeigt den Applikator gemäß Figur 5 bei betätigtem Abzugshebel.
Die Figuren 9a bis 9e zeigen, wie der erfindungsgemäße Applikator nach dem Reinigen wieder mit dem Schlauch verbunden und vollständig zusammengebaut wird.
Die Figur 10 zeigt den Applikator 1 und einen Medikamentenzerstäuber 33 in auseinandergebautem Zustand.
Die Figur 11 zeigt den Medikamentenzerstäuber 33 in explodierter Darstellung.
Die Figur 12 zeigt einen Mittellängsschnitt durch die von Figur 10 gezeigten Gegenstände.
Die Figur 13 zeigt den Medikamentenzerstäuber mit eingekoppeltem Kupplungsrohr des Applikators.
Die Figur 14 zeigt ein Schnellkupplungssystem für eine Sprühlanze und dergleichen.
Die Figur 15 zeigt eine Ablage für den Medikamentenzerstäuber schräg von der Seite her gesehen, von der her der Applikator angekurbelt wird.
Die Figur 16 zeigt die Ablage gemäß Figur 15 von oben.
Die Figur 17 zeigt die Ablage gemäß Figur 15 von der Seite.
Die Figur 18 zeigt einen Mittellängsschnitt durch einen Medikamentenzerstäuber, der in der Ablage gemäß Figur 15 gehalten wird.
Die Figur 19 zeigt einen Medikamentenzerstäuber mit einem abweichend gestalteten Kupplungssystem im Mittellängsschnitt vor dem Einsetzen des Applikators 1.
Die Figur 20 zeigt einen Medikamentenzerstäuber mit einem abweichend gestalteten Kupplungssystem gem. Fig. 19 im Mittellängsschnitt nach dem Einsetzen des Applikators 1.
Die Figur 21 zeigt einen Medikamentenzerstäuber mit einem abweichend gestalteten Kupplungssystem im Mittellängsschnitt vor dem Einsetzen des Applikators 1.
Die Figur 22 zeigt einen Medikamentenzerstäuber mit einem abweichend gestalteten Kupplungssystem gem. Fig. 21 im Mittellängsschnitt nach dem Einsetzen des Applikators.
Die Figur 23 zeigt eine Seitenansicht eines Applikators mit einem Strahlrohr mit aufgesetztem Spritzschutz.
Die Figur 24 zeigt den Applikator gemäß Figur 23 im Mittellängsschnitt.
Die Figur 25 zeigt eine Seitenansicht eines Applikators mit einem Strahlrohr mit integral angeformtem Spritzschutz.
Die Figur 26 zeigt den Applikator gemäß Figur 25 im Mittellängsschnitt.
Die Figur 27 zeigt einen Applikator mit einem aufgestellten weichen oder harten Aufsatz für die Durchführung des Politzer-Manövers.
Die Figur 28 zeigt die Figur 27 Mittellängsschnitt.
Die Abfolge gemäß der Figur 29 zeigt das Ankuppeln eines Applikators an den Medikamentenzerstäuber und die anschließende Zerstäubung.
Figur 30 zeigt eine Alternative, bei der das Handstück und der Dosator nicht miteinander verschraubt, sondern miteinander verstiftet sind.

### AUSFÜHRUNGSBEISPIELE

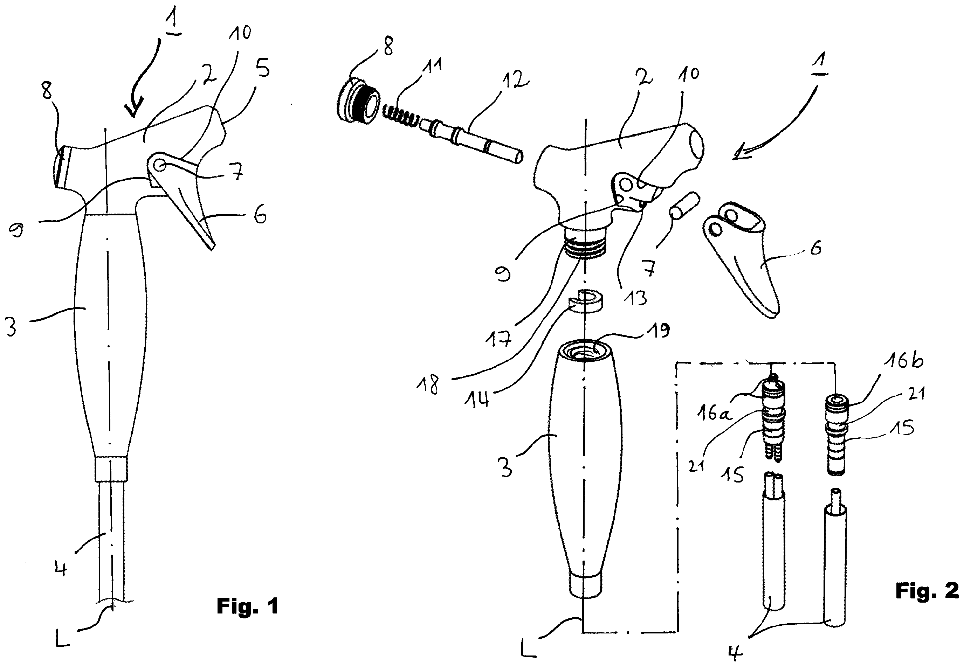

Die Hauptkomponenten des erfindungsgemäßen Applikatorsystems sind gut anhand der Figur 1 zu erkennen.

Das Applikatorsystem basiert auf einem Applikator 1, der seinerseits einen Dosator 2 umfasst. Dieser Dosator ist mit einem Handstück 3 verbunden, das seinerseits den Versorgungsschlauch 4 hält. Über den Versorgungsschlauch 4 wird das zu applizierende Fluid an den Dosator 2 herangeführt.

Das zu applizierende Fluid wird über die Mündung 5 des Dosators 2 ausgegeben, sobald der Dosierhebel 6 des Applikators betätigt wird. An der Mündung 5 des Dosators 2 kann ein hier nicht gezeigter Applikatoraufsatz befestigt sein, etwa ein Rohr mit einer Politzer-Olive, eine Kanüle oder ein Rohr, das an einen Medikamentenzerstäuber anzuschließen ist und diesen mit dem benötigten Fluid speist.

Vorzugsweise ist der Dosator 2 dabei so konstruiert, dass die Menge des applizierten Fluides darüber gesteuert werden kann, wie weit der Abzug 6 des Applikators 1 gezogen wird. Jedenfalls wird die Applikation, d. h. die Ausgabe des zu applizierenden Fluides, über die Mündung 5 durch Ziehen des Abzugs 6 ausgelöst.

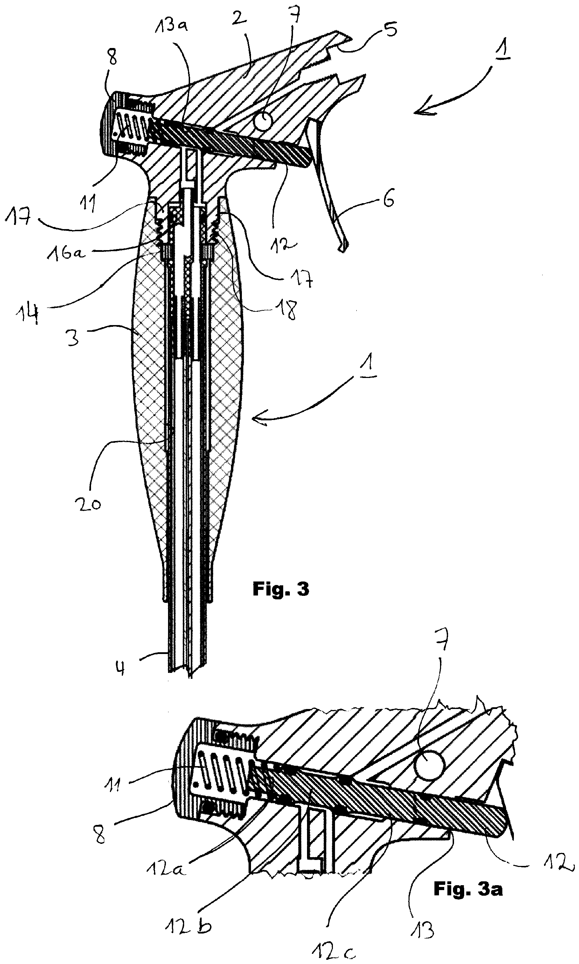

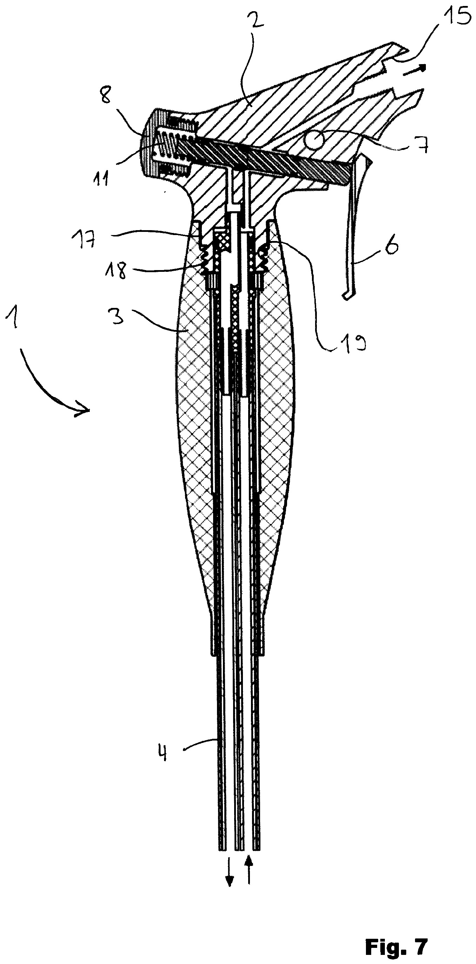

Zu diesem Zweck ist der Abzug 6 mittels des Achsstifts 7 zwischen zwei Anschlägen 9 und 10 hin und her schwenkbar an dem Dosator 2 befestigt. Wegen der diesbezüglichen Einzelheiten wird schon an dieser Stelle auf die Figuren 3 und 7 verwiesen.

Vorzugsweise ist der Achsstift 7 derart in dem Dosator 2 gehalten, meist durch Presssitz, dass er herausgedrückt werden kann, um den Abzug 6 zum Zwecke des Autoklavierens vom Dosator abnehmen zu können.

Das Handstück 3 ist typischerweise so ausgeführt, dass es einen Handgriff bildet, mit dem der Applikator 1 bequem so gegriffen werden kann, dass der Abzug 6 mit dem Zeigefinger der den Applikator 1 haltenden Hand bedient werden kann. Zu diesem Zweck weist das Handstück 3 in Richtung seiner Längsachse L vorzugsweise eine Länge auf, die zumindest die Breite einer Hand beträgt. Vorzugsweise beträgt die Länge mindestens 6 cm, idealerweise mindestens 8 cm. Bei Einhaltung einer solchen Mindestlänge kann der Applikator nicht nur gut gehandhabt werden. Vielmehr ist die Einhaltung einer solchen Länge besonders dann ausgesprochen günstig, wenn der Dosator 2 und das Handstück 3 miteinander verschraubt werden, weil dann an dem Handstück 3 beim Lösen vom und beim Festziehen am Dosator 2 bequem mit bloßer Hand das benötigte Drehmoment aufgebracht werden kann, auch wenn das im wesentlichen rotationssymmetrische Handstück 3, anders als der typischerweise unrunde Dosator 2, keinen Hebelarm bietet.

Zu sehen ist in der Figur 1 noch die Ventilverschlusskappe 8, deren Funktion gleich noch näher erläutert wird.

Ein genaueres Bild von den einzelnen Hauptkomponenten des Applikatorsystems gewährt die Figur 2, bei der es sich um eine Explosionszeichnung des Applikators 1 gemäß Figur 1 handelt.

Gut zu erkennen sind hier wieder der Dosator 2 und das Handstück 3 sowie der Abzug 6, der Achsstift 7 und die Vertiefung im Dosator 2, die die beiden Anschläge 9 und 10 ausbildet, zwischen denen der Abzug 6 hin und her bewegt werden kann.

Die Ventilverschlusskappe 8 ist in abgeschraubtem Zustand dargestellt. Gut zu erkennen ist, wie sich das Federelement 11 bevorzugt an der Innenseite der Ventilverschlusskappe 8 abstützt, um eine federnde Hin- und Her-Bewegung des Ventilschiebers 12 in der entsprechenden Ventilschieberbohrung 13a des Dosators 2 zu ermöglichen. Knapp noch zu erkennen ist die Mündung 13 der Ventilschieberbohrung 13a, über die der Ventilschieber 12 auf der Seite des Abzugs 6 aus dem Dosator 2 austritt, um sich gegen den Abzug 6 auf dessen Innenseite abzustützen. Sobald man sich dies vor Augen geführt hat, erkennt man, dass sich der Ventilschieber 12 gegen die Vorspannung des Federelements 11 in der Ventilschieberbohrung 13a hin und her bewegen lässt, indem man den Abzug 6 mehr oder minder stark zieht.

Sodann zeigt die Figur 2, dass der Dosator 2 eine Kupplungsmuffe 17 ausbildet, die vorzugsweise einstückig-integraler Bestandteil des Dosators 2 ist. Wie man sieht, trägt diese Kupplungsmuffe 17 im vorliegenden Fall auf ihrer Außenseite ein Bolzengewinde 18. Mittels dieses Bolzengewindes 18 kann der Dosator 2 über ein im Handstück 3 ausgebildetes Mutterngewinde 19 mit dem Handstück 3 verschraubt werden. Bei entsprechender Ausführung der Gewindesteigung ist diese Verschraubung werkzeuglos herstellbar und lösbar. Dadurch lässt sich der Applikator 1 schnell zerlegen, wenn er zu Reinigungszwecken autoklaviert werden soll oder auf sonstige Art und Weise gereinigt werden muss.

Gut anhand der Figur 2 zu erkennen ist auch das Halteorgan 14, auf dessen Funktion später noch näher einzugehen ist.

Im unteren Bereich der Figur 2 erkennt man zwei alternativ zu verwendende Versorgungschläuche 4, die jeweils an einem Kupplungsteil 15 fluiddicht festgelegt sind. Das Kupplungsteil 15, das mit dem linken dieser beiden Versorgungschläuche 4 verbunden ist, bildet einen zweistufigen Kupplungsnippel 16a aus.

Das Kupplungsteil 15, das mit dem rechten dieser beiden Versorgungsschläuche 4 verbunden ist, bildet einen einstufigen Kupplungsnippel 16b aus. Der Zweck dieser ein- und zweistufigen Kupplungsnippel wird später noch näher erläutert.

Die Figur 3 zeigt einen Achsschnitt durch einen Applikator 1 des erfindungsgemäßen Applikatorsystems. Hier ist das Zusammenspiel der bereits geschilderten Komponenten gut zu erkennen.

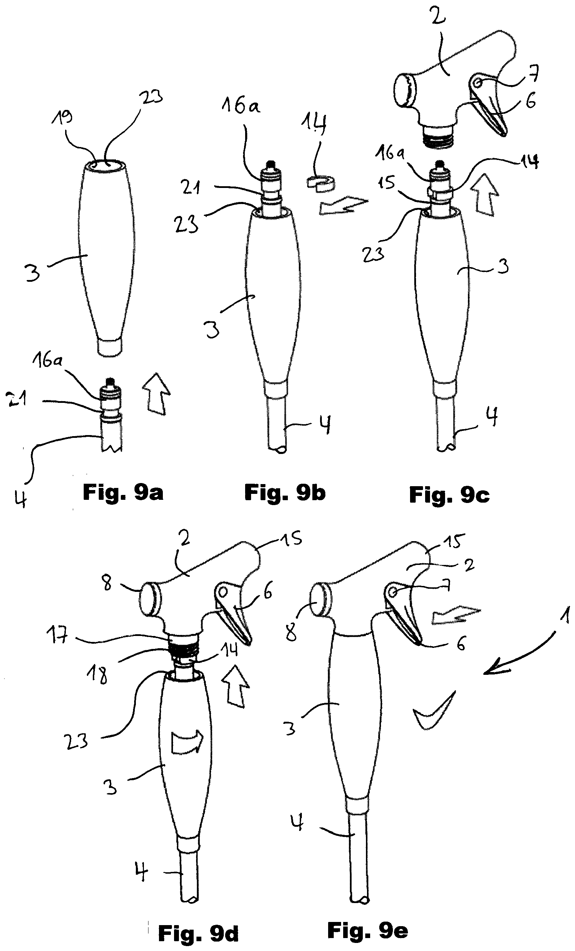

Insbesondere die gemeinsame Betrachtung der Figuren 3 und 9 veranschaulicht den erfindungsgemäß besonders einfachen Auseinander- und Zusammenbau des Applikators 1.

Das Handstück 3 ist auf ganzer Länge von der Bohrung 20 durchdrungen, die einen Abschnitt des Versorgungsschlauchs 4 und das mit ihm verbundene Kupplungsteil 15 aufnimmt.

Der Versorgungsschlauch 4 und das mit ihm verbundene Kupplungsteil 15 sind von der dem Dosator 2 abgewandten Seite her in das Handstück 3 eingeführt worden, vorzugsweise so weit, bis das Kupplungsteil 15 auf der dem Dosator 2 zugewandten Seite des Handstücks 3 wieder herausgeragt hat. Dann ist das hier vorzugsweise als C-förmige Scheibe ausgebildete Halteorgan 14 quer zur Längsachse L des Schlauchs in die Haltenut 21 des Kupplungsteils 15 eingeschoben worden. Hierdurch bildet das C-förmig ausgebildete Halteorgan eine Art Kragen an dem Kupplungsteil 15.

Vorzugsweise als nächstes ist der vom Kupplungsteil 15 ausgebildete Kupplungsnippel 16a in die Kupplungsmuffe 17, die hier am Dosator 2 ausgebildet ist, eingeschoben worden.

Anschließend sind das Handstück 3 und der Dosator 2 miteinander gekoppelt worden, im vorliegenden Fall vorzugsweise dadurch, dass das Handstück 3 mit dem Mutterngewinde 19 seiner Einschrauböffnung 23 auf das Bolzengewinde an der Außenseite der Kupplungsmuffe 17 des Dosators 2 aufgeschraubt worden ist. Dabei kommt das den Kragen bildende Halteorgan 14 in dem hierfür vorgesehenen Sitz 22 des Handstücks 3 zu liegen. Vorzugsweise wird es dabei formschlüssig zwischen dem Handstück 3 an seiner einen Stirnseite und dem Dosator 2 an seiner anderen Stirnseite gehalten.

Der besagte formschlüssige Halt ist verspannungsfrei oder mit Spiel ausgeführt. Dadurch bleibt der Versorgungsschlauch 4 gegenüber dem Handstück 3 und dem Dosator 2 drehbar, was die Arbeit mit dem Applikator 1 erheblich erleichtert, da sich keine Spannung durch Verdrehung des Versorgungsschlauchs 4 aufbauen kann.

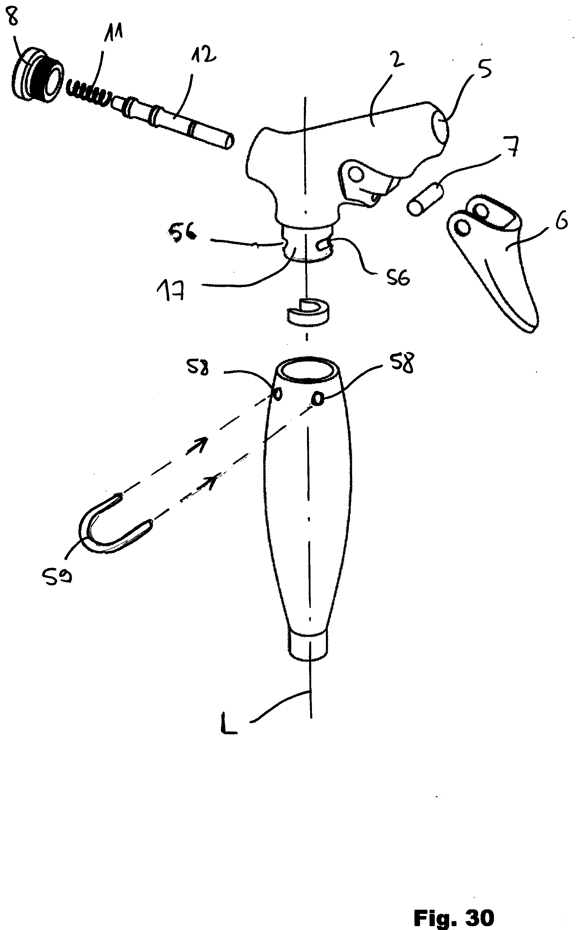

Alternativ ist denkbar, das Handstück 3 und den Dosator 2 miteinander zu verstiften, anstatt die bevorzugte Gewindeverbindung zu wählen, so, wie das Fig. 30 zeigt. Der Kupplungsflansch besitzt bei dieser Variante z. B. zwei Stiftaufnahmenuten 56. Sobald die Kupplungsmuffe 17 in das Handstück 3 eingeschoben worden ist, wird durch die Stiftdurchschuböffnungen 58 ein beispielsweise U-förmig ausgebildeter Sicherungsstift 59 eingeschoben. Er legt sich in die Stiftaufnahmenuten 56 der Kupplungsmuffe 17 ein und tritt dann beispielsweise durch in Fig. 30 nicht erkennbare Stiftdurchschuböffnungen 58 auf der dem Betrachter abgewandten Seite des Handstücks wieder aus diesem aus. Durch den Formschluss des Sicherungsstifts 59 mit den Stiftaufnahmenuten 56 sind der Dosator 2 und das Handstück 3 sicher aneinander festgelegt.

Insgesamt ist also festzuhalten, dass es vorzugsweise so ist, dass die Kupplungsmuffe 17 des Dosators 2 in vollständig montiertem Zustand in radialer Richtung (senkrecht zur Längsachse L) zumindest teilweise, idealerweise überwiegend zwischen dem Handstück 3 und dem Kupplungsnippel 16a bzw. 16b angeordnet ist. Dabei wird die Kupplungsmuffe 17 vollständig in der Einschrauböffnung 23 des Handstücks 3 aufgenommen.

Anhand dieser Schilderungen ist erkennbar, dass sich das Applikatorsystem sehr einfach und sicher zerlegen lässt, um es in irgendeiner Form zu reinigen. Man führt nur die oben beschriebenen Vorgänge in umgekehrter Reihenfolge aus. D. h. man schraubt den Dosator 2 vom Handstück 3 ab, verschiebt dann das Kupplungsteil 15 so lange gegenüber dem Handstück 3, bis man das Halteorgan 14 ungehindert in radialer Richtung abziehen kann, um dann das Kupplungsstück 15 mitsamt dem Versorgungsschlauch 4 zu der dem Dosator 2 abgewandten Seite des Handstücks 3 aus diesem herauszuziehen.

Nun können der Dosator 2 und das Handstück 3 sehr einfach gereinigt werden.

Vorzugsweise schraubt man zu diesem Zweck noch die Ventilverschlusskappe 8 ab, um dann den Ventilschieber 12 vollständig aus der Ventilschieberbohrung 13a des Dosators 2 herauszuziehen, vom Dosator 2 zu trennen und so einer perfekten Reinigung zugänglich zu machen.

Anhand der Figur 3 lässt sich auch recht gut erläutern, was es mit dem zweistufigen Kupplungsnippel 16a auf sich hat. Wie man anhand der Figur 3 sieht, ist der Versorgungsschlauch 4 in diesem Fall zweiflutig ausgeführt, d. h. er besitzt einen ersten Kanal, über den dem Dosator Fluid zugeführt werden kann, und einen davon getrennten zweiten Kanal, über den Fluid vom Dosator abgeführt werden kann. Meist führt ein zweiflutiger Versorgungsschlauch ein Fluid in Gestalt einer Flüssigkeit, während ein nur einflutiger Versorgungsschlauch ein gasförmiges Fluid führt.

Wie man sieht, weist der Kupplungsnippel 16a einen ersten Nippelabschnitt 24 auf, der einen großen Außendurchmesser besitzt. Dieser erste Nippelabschnitt 24 liegt dicht gegen einen ersten Muffenabschnitt 26 an. Stirnseitig bildet der erste Nippelabschnitt 24 mindestens eine Mündung 28, die fluidisch mit dem Dosator 2 in Verbindung steht. Sodann weist der Kupplungsnippel 16a einen zweiten Nippelabschnitt 25 auf, der einen gegenüber dem ersten Nippelabschnitt 24 verringerten Außendurchmesser besitzt. Dieser zweite Nippelabschnitt 25 liegt dicht gegen einen zweiten Muffenabschnitt 27 an. Stirnseitig bildet der zweite Nippelabschnitt 25 mindestens eine Mündung 29, die fluidisch mit dem Dosator 2 in Verbindung steht. Auf diese Art und Weise kann permanent Fluid durch den Dosator zirkulieren, auch dann, wenn nicht appliziert wird. Das verhindert, dass an irgendeiner Stelle des Dosators 2 ruhendes Fluid ansteht, in dem sich unter ungünstigen Umständen eine Verkeimung bilden bzw. ungestört anwachsen kann.

Zu guter Letzt ist anhand der Figur 3 im Zusammenspiel mit der Figur 3a, die eine entsprechende Ausschnittvergrößerung zeigt, noch die Funktionsweise und die bevorzugte Ausgestaltung des Ventilschiebers 12 zu erläutern.

Vorzugsweise besitzt der Ventilschieber 12 auf seiner dem Federelement 11 zugewandten Seite einen Führungsabschnitt 12a. Auf diesen ist das hier als Schraubenfeder ausgeführte Federelement 11 aufgeschoben. Zusätzlich besitzt der Ventilschieber 12 einen Brückenabschnitt 12b, der beidseitig von zwei Dichtungsabschnitten 12c flankiert wird. Daran schließt sich ein Fortsetzungsabschnitt 12d des Ventilschiebers an. Der Fortsetzungsabschnitt 12d wird, abgedichtet durch die Mündung 5 des Dosators 2, nach außen geführt und liegt gegen den Abzug 6 an.

Vom ersten Muffenabschnitt 26 und vom zweiten Muffenabschnitt 27 aus führen zwei Verbindungskanäle 30 und 31 in den Dosator hinein, die bei unbetätigtem Abzugshebel 6 in den Bereich des Ventilschiebers 12 münden, der den Brückenabschnitt 12b des Ventilschiebers 12 bildet. Auf diese Art und Weise ist auch bei ungezogenem Abzugshebel 6 eine Zirkulation möglich.

Sobald der Abzugshebel 6 gezogen wird und sich der dem Abzugshebel am nächsten liegende Dichtungsabschnitt 12c daraufhin über den dem Abzugshebel am nächsten liegenden Verbindungskanal 31 hinweg bewegt, entsteht eine fluidische Verbindung zwischen dem besagten Verbindungskanal und dem Ausgabekanal 32, der zur Mündung 5 führt, vergleiche Figur 7.

Jedenfalls dann, wenn der Abzugshebel 6 vollständig gezogen ist, ist es vorzugsweise so, dass der besagte Dichtungsabschnitt 12c die fluidische Verbindung zwischen den Verbindungskanälen 30 und 31 unterbricht, sodass nun der gesamte dem Dosator 2 über den Verbindungskanal 31 zugeführte Fluidstrom über den Ausgabekanal 32 und die Mündung 5 abgegeben wird.

Es leuchtet leicht ein, dass die Konstruktion vorzugsweise so ausgelegt sein kann, dass der Volumenstrom des ausgegebenen Fluids dadurch gesteuert werden kann, wie weit der besagte Dichtungsabschnitt 12c über den Verbindungskanal 31 bzw. dessen Mündung hinweggeschoben worden ist.

Wenn der Dichtungsabschnitt 12c fast vollständig über dem Kanal 31 bzw. seiner Mündung liegt, sodass er diesen fast vollständig abdeckt, dann wird nur ein kleiner Volumenstrom ausgegeben. Der ausgegebene Volumenstrom wird umso größer, je mehr der Dichtungsabschnitt 12c den Kanal 31 bzw. dessen Mündung in Richtung hin zu dem Federelement 11 passiert hat.

Festzuhalten ist noch, dass der zweite der Dichtungsabschnitte 12c die Funktion übernimmt, den Ventilschieber 12 zur Seite des Federelements 11 hin abzudichten, sodass dieses und seine Aufnahmekammer nicht mit dem zu applizierenden bzw. zurücklaufenden Fluid in Kontakt kommen und sich hier auch kein "Totbereich" ausbildet, in dem Fluid längere Zeit stehen bleiben kann und zu verkeimen droht.

Besonders günstig ist es, wenn das Applikatorsystem auch einen optional ankuppelbaren Zerstäuber bzw. Medikamentenzerstäuber besitzt.

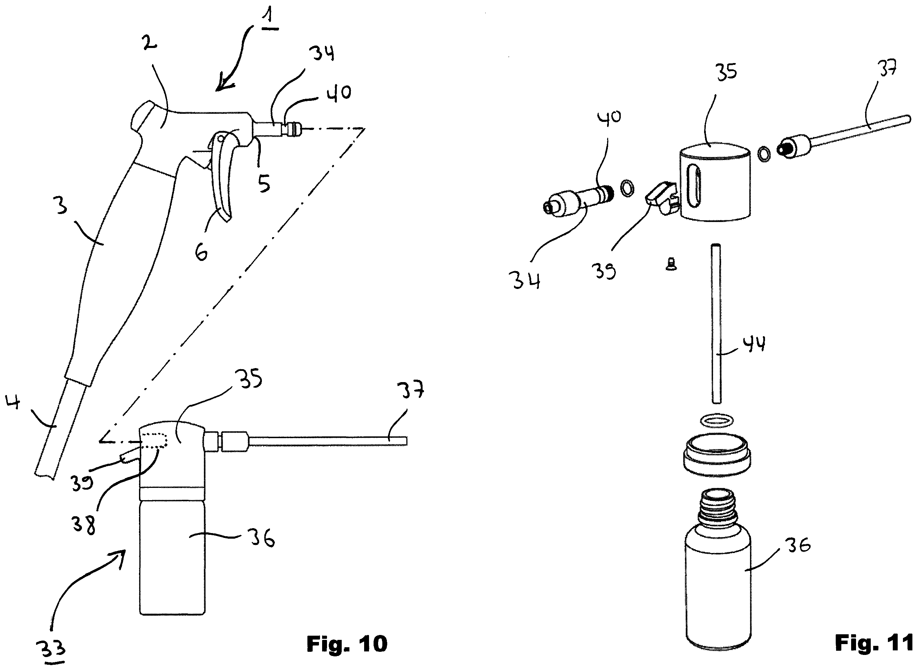

Die Figur 10 zeigt eine Explosionszeichnung eines solchen Applikatorsystems 1 aus einem erfindungsgemäßen Applikator und einem daran ankuppelbaren Medikamentenzerstäuber 33.

Wie man sieht, ist der Applikator 1 hier mit einem Kupplungsrohr 34 ausgerüstet, das in seine Mündung 5 dichtend eingesetzt ist. Die übrigen Bestandteile des Applikators 1 sind bereits beschrieben worden und werden daher hier nicht nochmals näher angesprochen.

Der Medikamentenzerstäuber 33 besteht aus einem Zerstäuberoberteil 35, das auf einen Vorratsbehälter 36 aufgesetzt und dicht mit diesem verbunden ist. Von dem Zerstäuberoberteil 35 geht eine Zerstäuberlanze 37 ab, über die das dem Medikamentenzerstäuber 33 vom Applikator 1 her zugeführte Fluid zusammen mit dem in ihm zerstäubten Medikament bzw. anzuwendenden Stoff ausgegeben wird.

Wie der Medikamentenzerstäuber funktioniert, wird gleich noch näher beschrieben. Wichtig ist, dass der Medikamentenzerstäuber 33 eine Kupplungsaufnahme 38 besitzt. Angrenzend an die Kupplungsaufnahme 38 ist ein Sperrhebel 39 schwenkbar gelagert. Der Applikator 1 kann mit seinem Kupplungsrohr 34 in die Kupplungsaufnahme 38 eingeschoben werden. Das Kupplungsrohr, das in seinem freien Ende zweckmäßigerweise mit einer Schnurdichtung ausgestattet ist, kann so lange in die Kupplungsaufnahme 38 eingeschoben werden, bis der Sperrhebel 39 in die Nut 40 des Kupplungsrohrs 34 eingreift und eine formschlüssige Verbindung zwischen dem Kupplungsrohr 34 und der Kupplungsaufnahme 38 herstellt, so, dass der Applikator 1 und der Medikamentenzerstäuber 33 eine Einheit werden.

Um die Kupplung zu lösen und den Medikamentenzerstäuber 33 vom Applikator 1 abnehmen zu können, muss hier nur der Sperrhebel 39 niedergedrückt werden, im vorliegenden Beispielsfall nach oben. Dann können der Applikator 1 bzw. dessen Kupplungsrohr 34 leicht abgenommen werden.

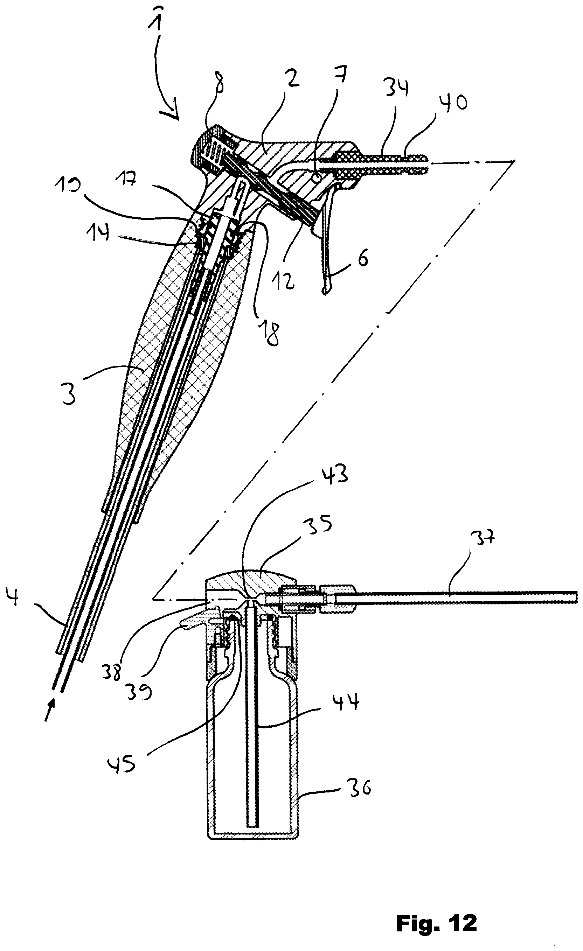

Die Einzelheiten des Medikamentenzerstäubers zeigen dessen Figuren 11 und 12.

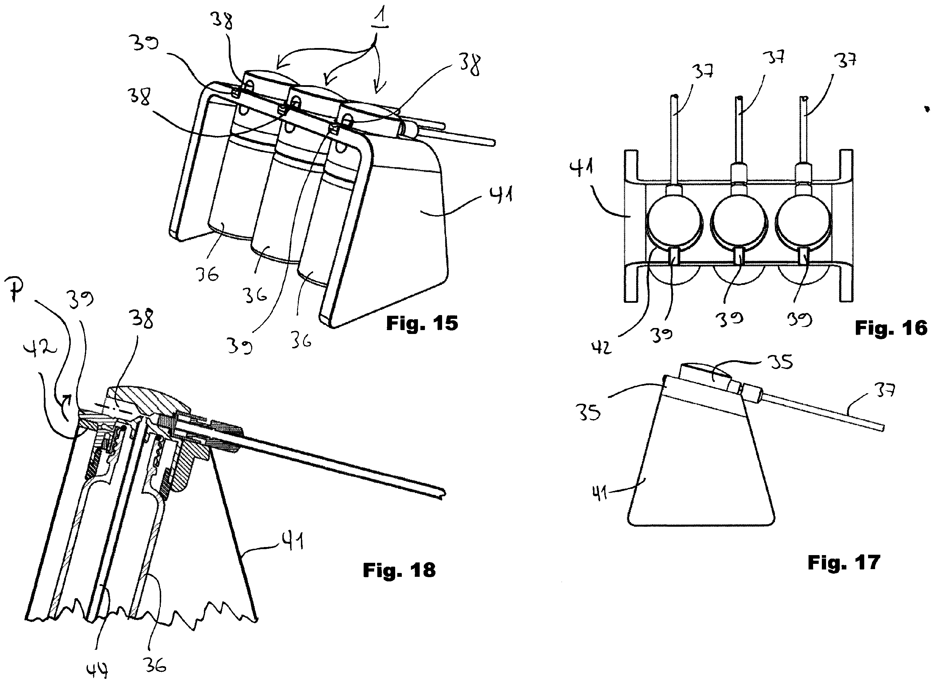

Schon an dieser Stelle ist festzuhalten, dass zu dem Applikatorsystem vorzugsweise auch eine Ablage 41 für den oder die Medikamentenzerstäuber 33 gehört, wie sie die Figuren 15 bis 18 zeigen. Die Ablage besitzt eine Aufnahme 42 zum Halten mindestens eines Medikamentenzerstäubers 33, auch in abgekoppeltem Zustand.

Die Aufnahme 42 ist so gestaltet, dass der Sperrhebel 39 unter dem Eigengewicht des in der Aufnahme 42 hängenden Medikamentenzerstäubers niedergedrückt wird und sich dadurch (im vorliegenden Fall) ein Stück weit in Richtung des Uhrzeigersinns bewegt, vgl. den Pfeil P in der Fig. 18. Dadurch gibt der Sperrhebel 39 die Kupplungsaufnahme 38 völlig frei.

Gleichzeitig ist die Ablage 41 vorzugsweise so gestaltet, dass sie den Medikamentenzerstäuber leicht zur Vertikalen geneigt hält, sodass seine Kupplungsaufnahme 38 in betriebsbereitem Zustand und in Bezug auf die Vertikale oberhalb der Zerstäuberlanze 37 zu liegen kommt. Auf diese Art und Weise, aber auch sonst, kann der Medikamentenzerstäuber 33 vom Anwender leicht mit dem Applikator 1 aufgenommen werden, mit dem er gerade arbeitet bzw. arbeiten will. Das besagte Aufnehmen erfolgt, indem man den Applikator 1 an den Zerstäuber heranführt und dann das Kupplungsrohr 34 des Applikators 1 ungehindert durch den Sperrhebel 39 in die Kupplungsaufnahme 38 einführt. In dem Moment, in dem man dann den so gegriffenen Medikamentenzerstäuber 33 aus der Ablage 41 heraushebt, bewegt sich der Sperrhebel 39 unter dem Einfluss seiner elastischen Vorspannung ein Stück weit im Gegenuhrzeigersinn, sodass er mit der Nut 40 des Kupplungsrohrs 34 in Eingriff kommt und automatisch eine feste Verbindung hergestellt ist.

Beim Wiederablegen des Medikamentenzerstäubers 33 mithilfe des Applikators 1 in der Ablage 41 kommt es von allein, unter dem Einfluss des Eigengewichts des Medikamentenzerstäubers, wieder zum Niederdrücken des Sperrhebels 39 und damit zum automatischen Abkuppeln. Derartige Systeme sind besonders einfach zu bedienen, auch mit behandschuhten Händen und/oder Händen, die durch zu applizierende Substanzen oder im Zuge einer Untersuchung am Körper schlüpfrig geworden sind.

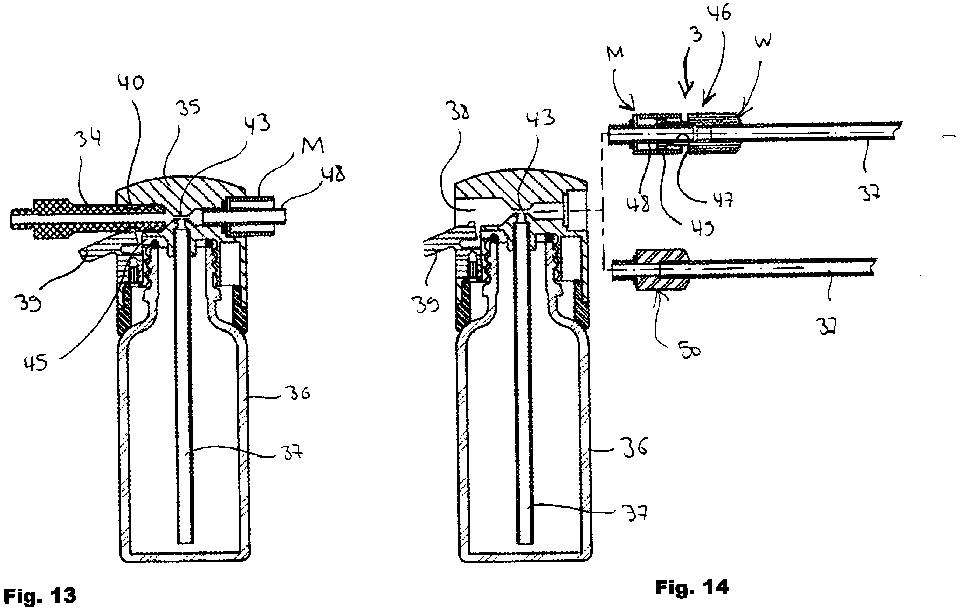

Die Funktionsweise, wie der Medikamentenzerstäuber 33 an den Applikator formschlüssig oder alternativ auch reibschlüssig (dazu sogleich) angekuppelt wird, lässt sich auch gut anhand der Figuren 13 und 14 nachvollziehen.

Anhand der Fig. 13 und 14 ist auch der Zerstäuber gut zu beschreiben. Bei dem Zerstäuber handelt es sich bevorzugt um einen Venturizerstäuber. Zu diesem Zweck verengt sich der Fluidweg innerhalb des Zerstäubers unmittelbar nach dem Ende des Kupplungsrohrs 34 zu einer Venturidüse 43.

Von unten her, also quer zur Haupt-Strömungsrichtung, mündet ein Steigrohr 44 in die Seitenwand der Venturidüse 43 ein. Das Steigrohr endet mit seinem anderen Ende im Vorratsbehälter 36. Durch den in der Venturidüse entstehenden Unterdruck wird Fluid aus dem Vorratsbehälter 36 angesaugt und dort, wo das Steigrohr in die Venturidüse ausmündet, vernebelt bzw. verwirbelt und damit zerstäubt.

Wie man sieht, ist vorzugsweise unmittelbar im Bereich des Sperrhebels 39 eine Druckausgleichsöffnung 45 vorgesehen. Durch sie kann aus der Umgebung Luft in den Vorratsbehälter 36 nachfließen und so verhindern, dass dort ein Vakuum entsteht, was schließlich das weitere Ansaugen und Vernebeln gefährden würde.

Ebenfalls gut anhand der Fig. 14 lässt sich das Schnellkupplungssystem erklären, das erfindungsgemäß zum Einsatz kommt, um einfach, schnell und sicher verschiedene, austauschbare Applikator- und/oder Zerstäubervorsätze zu befestigen.

Vorzugsweise ist das Schnellkupplungssystem nach Art eines sogenannten Luer-Lock-Verschlusses 46 gestaltet, wie das in Fig. 14 dargestellt ist. Der Luer-Lock-Verschluss besteht aus einem weiblichen Teil W, der hier vorzugsweise an dem Vorsatz ausgebildet ist, der im vorliegenden, nur exemplarischen Fall die Gestalt einer Zerstäuberlanze 37 hat.

Der weibliche Teil W besitzt eine - vorzugsweise hohlkegelig, ansonsten zylindrisch ausgebildete - Innenaufnahme 47, die über den vorzugsweise außenkegeligen, ansonsten zylindrisch ausgebildeten Zapfen 48 des männlichen Teils M geschoben wird, sodass sich eine Abdichtung einstellt.

Der männliche Teil M besitzt zusätzlich, konzentrisch zu seinem Zapfen und zumindest überwiegend um diesen herum angeordnet, noch eine zylindrische Hohlschürze 49 mit einem Innengewinde. In dieses wird das entsprechende Außengewinde am weiblichen Teil W eingedreht, sodass ein unbeabsichtigtes Trennen der Verbindung in Richtung der Längsachse vereitelt wird. Gesagt sei noch, dass der Zapfen 48 des männlichen Teils M vorzugsweise länger ist als dessen Hohlschürze 49, sodass erst die gegeneinander dichtenden Teile eingefädelt werden können, bevor die sichernde Gewindeverbindung hergestellt wird.

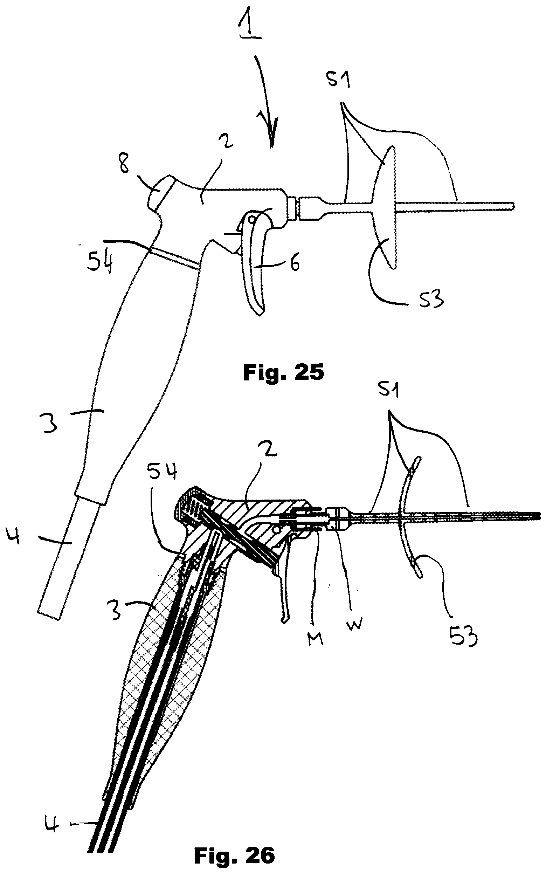

Wie man gut anhand der Fig. 26 sieht, kann ein solches Schnellkupplungssystem auch dazu verwendet werden, um - ohne Verwendung eines Zerstäubers 33 - unmittelbar an den Applikator selbst einen Applikatorvorsatz zu befestigen.

Gegebenenfalls reicht auch eine einfache Luer-Verbindung aus, wie sie - mit der Bezugsziffer 50 markiert - in Fig. 14 als Alternative dargestellt ist.

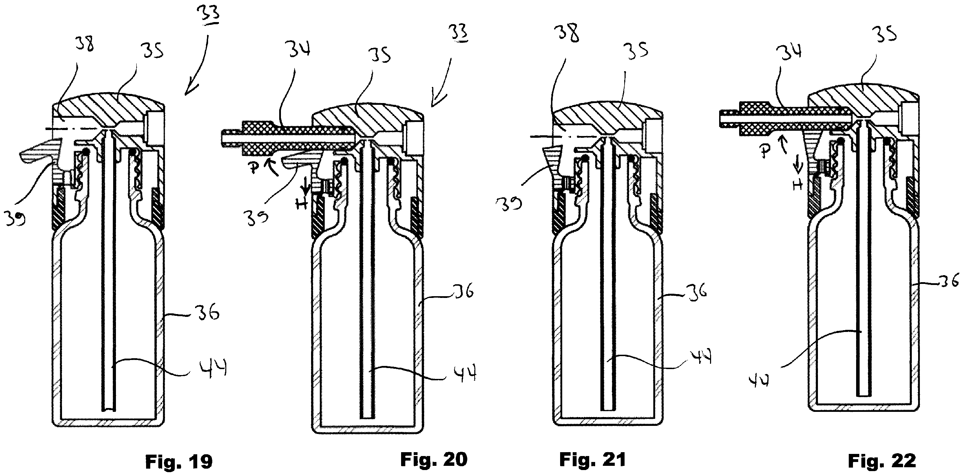

Eine alternative Ausgestaltung zeigen die Figuren 19 und 20.

Für diese Figuren gilt das zuvor Gesagte sinngemäß, soweit sich aus dem nachfolgend explizit beschriebenen Unterschied nicht etwas anderes ergibt.

Der einzige Unterschied liegt hier in der Gestaltung des Sperrhebels. Der hier verwendete Sperrhebel sichert nämlich nicht formschlüssig.

Das sieht man bei einem Vergleich der Figuren 19 und 20. Der Sperrhebel 39 besteht aus einem Material, das gegenüber dem Kupplungsrohr 34 des Applikators 1 hinreichend große Reibungskräfte entfaltet, vorzugsweise kommt ein TPE oder ein Elastomer zum Einsatz. Idealerweise ist das verwendete Material in sich komprimierbar, sodass es sich beim Kupplungsvorgang zusammendrücken lässt.

Bei leerer Kupplungsaufnahme 38 ragt der Sperrhebel 39 in den ansonsten lichten Querschnitt der Kupplungsaufnahme 38 hinein, steht hier "im Wege", vgl. Fig. 19. In die zunächst leere Kupplungsaufnahme 38 (Figur 19) wird in horizontaler Richtung das Kupplungsrohr 34 bis auf Anschlag eingeschoben. Dabei wird der im Weg stehende Sperrhebel berührt, mitgenommen und ein Stück weit im Uhrzeigersinn entlang des Pfeils P gedreht. Vorzugsweise wird seine das Scharnier bildende Dünnstelle dabei auch komprimiert, sodass sich der oberhalb davon liegende Teil des Sperrhebels in Richtung des Pfeils H unter Aufbau elastischer Spannung in Richtung senkrecht zum Kupplungsrohr 34 nach unten bewegt (um einen kleinen Betrag).

Dadurch entsteht eine klemmende Wirkung zwischen dem Sperrhebel 39 und dem Kupplungsrohr 34. Aufgrund der im Zuge dessen entstehenden elastischen Vorspannung zwischen dem Sperrhebel 39 und dem Kupplungsrohr 34 wird das Kupplungsrohr 34 reibschlüssig in der Kupplungsaufnahme 38 festgehalten. Hierbei wird dieses reibschlüssige Festhalten vorzugsweise dadurch begünstigt, dass der Sperrhebel 39 so ausgestaltet ist, dass er beim Auftreten von Zugkräften an dem Kupplungsrohr 34 (in "Herausziehrichtung") die Tendenz hat, sich leicht im Gegenuhrzeigersinn zu bewegen, sich dabei um ein Stück im Gegenuhrzeigersinn "aufzurichten" und dadurch das Kupplungsrohr 34 erst einmal noch stärker zu bekneifen bzw. festzuspannen. Das vergrößert die Reibungskräfte zunächst und wirkt dem Losbrechen des Kupplungsrohrs 34 entgegen.

Das Kupplungsrohr trägt keine Rastnut, sondern hat eine glattzylindrische Mantelfläche und nur eine nicht kuppelnd wirkende Nut zur Aufnahme einer Schnurdichtung.

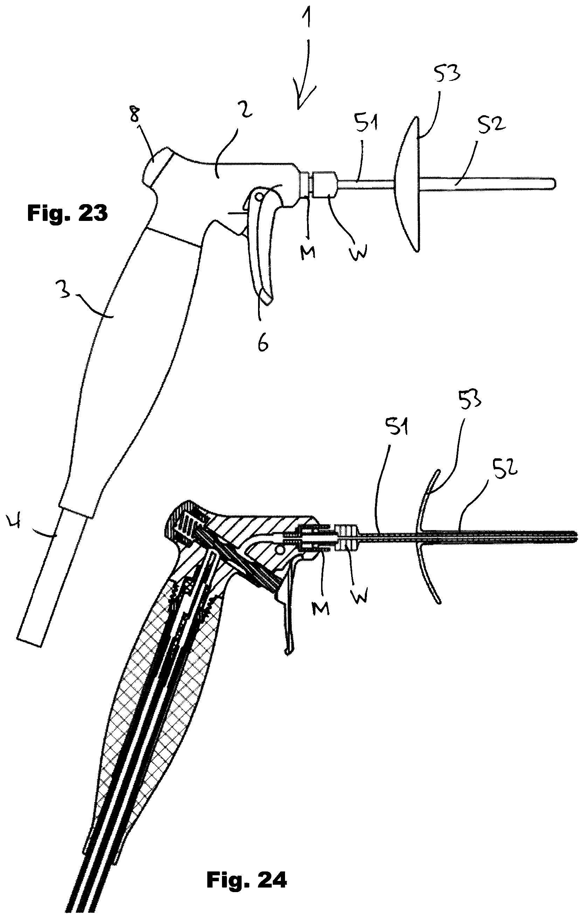

Die Figuren 21 und 22 zeigen eine ähnliche Alternativkonstruktion, die technisch auf dem gleichen Prinzip beruht wie die soeben beschriebene Alternativkonstruktion, aber allerdings keinen Hebelfortsatz zum manuellen Lösen aufweist, also keinen "Sperrhebel" im engeren Sinne. Diese Art der Kupplung kann man als "Stopfkupplung" bezeichnen. Sie wird durch beherztes "Hereinstopfen" des Kupplungsrohrs 34 in die Kupplungsaufnahme geschlossen und durch sein beherztes Herausziehen wieder gelöst. Die Figuren 23 und 24 zeigen optionales aufsteckbares Zubehör für den flüssigen Applikator.

An die Mündung 5 des Applikators ist hier eine Applikationsspitze 51 angekuppelt, die den Strahl generiert, mit dem gearbeitet wird. Zu diesem Zweck kann die schon oben näher beschriebene, nach Art einer Luer-Lock-Verbindung ausgestaltete Kupplung zum Einsatz kommen.

Erfindungsgemäß ist eine Applikationshülse 52 zum Überziehen und Abdecken der Applikationsspitze 51 vorgesehen. Hier wird vor jeder Behandlung eine neue Applikationshülse 52 über die Applikationsspitze 51 geschoben. So werden mit einem Minimum an Aufwand für jeden Patienten hygienische und saubere Kontaktflächen geschaffen, mithilfe der bevorzugt als Wegwerfartikel ausgebildeten Applikationshülse 52.

Die Applikationshülse 52 ist vorzugsweise so an die Applikationsspitze angepasst und bevorzugt innenkegelig, dass sie sich mit derartiger Reibung auf die bevorzugt außenkegelige Applikationsspitze 51 aufschieben lässt, dass sie sich bei der Applikation nicht unbeabsichtigt verschieben kann und dadurch ungewollt einen Teil der hygienisch zu schützenden Fläche freigibt. Auf ihrer dem Patienten zugewandten Stirnseite verengt sich die Applikationshülse 52 bevorzugterweise und deckt so auch die freie Stirnringfläche der Applikationsspitze 51 ab. Sie bildet hier zugleich einen "Überziehstopper", wenn nicht wegen der kegeligen Ausgestaltung unnötig.

Diese Hülse 52 kann zum Beispiel ein Tiefzieh- oder (bevorzugt) Spritzgussteil sein, welches nach der Behandlung entsorgt wird.

Idealerweise ist diese Hülse mit einem radial von ihr - vorzugsweise rundum um mehrere Zentimeter - abstehenden und idealerweise zum Patienten hin geneigten bzw. konkaven Spritzschutz bzw. Schild 53 versehen, der die frei im Bereich hinter der Applikationshülse 52 liegenden Bereiche des Applikators hygienisch abschirmt.

Die Figuren 25 und 26 zeigen eine ähnliche Anordnung, die sich von der Anordnung der Fig. 23 und 24 nur dadurch unterscheidet, dass der Spritzschutz bzw. Schild 53 integraler Bestandteil der Applikationsspitze 51 ist. Gerade hier ergibt daher die Verwendung einer nach Art eines Luer-Lock gestalteten Kupplung Sinn.

Besonders bemerkenswert an der Variante nach Figuren 25 und 26 ist der Signalring 54. Er wird vor dem Zusammenbau von Handstück 3 und Dosator 2 um die Kupplungsmuffe 17 herumgelegt und kommt dann dauerhaft zwischen dem Handstück 3 und dem Dosator 2 zu liegen. Zu dem erfindungsgemäßen System können beispielsweise mehrere solcher Ringe in unterschiedlichen Farben gehören, die vom Benutzer (bei Bedarf) leicht selbst eingebaut werden können. Das ist beim parallelen Einsatz mehrerer unterschiedlicher Applikatoren 1 von Vorteil, da der Benutzer dann schnell, unübersehbar und unverlierbar markieren kann, wie ein bestimmter Applikator im konkreten Fall konfiguriert ist.

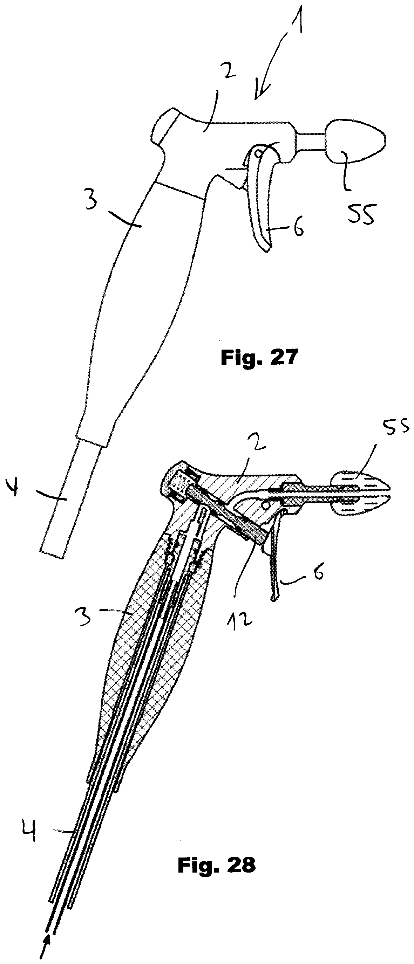

Die Figuren 27 und 28 zeigen optionales aufsteckbares Zubehör für den Applikator. Hier wird eine Olive für das Politzer-Manöver gezeigt. Diese kann hart oder besser weich sein, um sich an die Nase des Patienten anzupassen, mehr Komfort zu bieten und besser abzudichten. Dieses Teil ist leicht zu montieren und autoklavierbar.

### SONSTIGES ZU DER ERFINDUNG

Im Sinne allgemeingültiger Ausführungen ist, zum Teil zusammenfassend bzw. wiederholend, noch Folgendes zu der Erfindung zu sagen.

In Figur 1 ist der montierte Applikator dargestellt, dieser verfügt über ein gutes Handling, einen ergonomischen Griff und ist durch seine flächenbündigen Übergänge auch montiert gut zu reinigen. Der Versorgungsschlauch ist unsichtbar verbunden und ist dennoch drehbar gelagert.

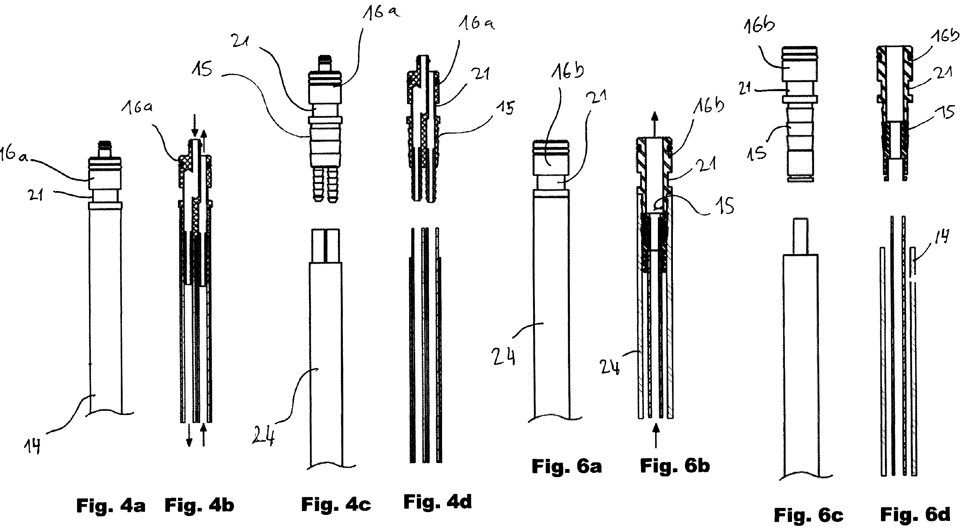

In Figur 2 sind alle erfindungsgemäßen Komponenten dargestellt. Die Figuren 3,4 und 7 zeigen den Aufbau des Applikators für die Anwendung mit flüssigen Medien. Eine Zirkulationsfunktion des Mediums und/oder des Desinfektions- bzw. Reinigungsmittels ist durch die kleinen Pfeile schematisch dargestellt.

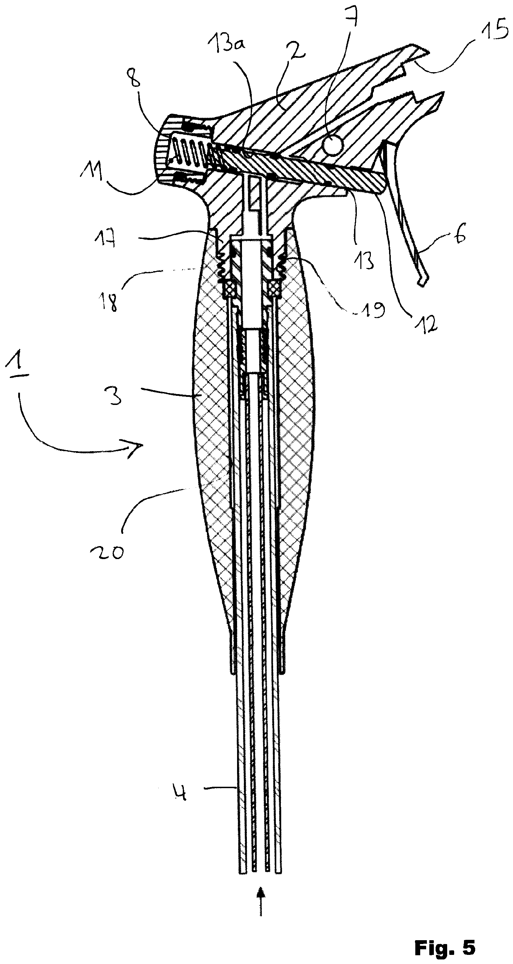

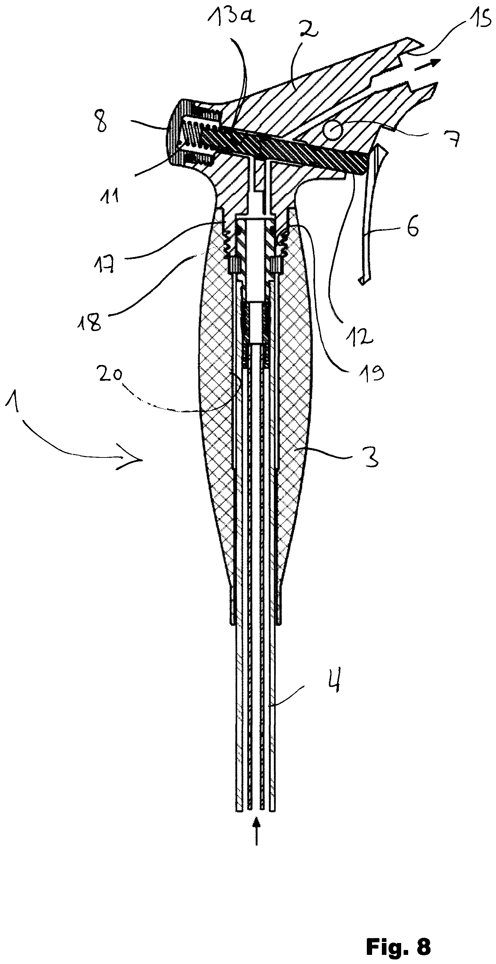

Die Figuren 5,6 und 8 zeigen den Aufbau des Applikators für die Anwendung mit gasförmigen Medien. Die Flussrichtung des Mediums ist durch die kleinen Pfeile schematisch dargestellt.

Die auch Adapter genannten Kupplungsteile 15 passen beide gleichermaßen in die Öffnung (Kupplungsmuffe) des Funktionsteiles (Dosators 2) und sind bevorzugt mit Dichtungen (Schnurdichtungen) versehen und rotationssymmetrisch und damit drehbar gelagert.

Der Zusammenbau des Applikators nach Figur 9 zum Applikator nach Figur 1 läuft demnach wie folgt ab:
Der Versorgungsschlauchanschluss (Versorgungsschlauch 4 mit daran fluiddicht montiertem Kupplungsteil 15) wird durch den Griff bzw. das Handstück 3 geführt. Der Klemmring bzw. das Halteorgan 14 wird auf die dafür vorgesehene Nut bzw. Haltenut 21 des Schlauchanschlusses bzw. Kupplungsteils 15 aufgeschoben und verhindert ein Zurückrutschen durch den Griff bzw. das Handstück 3. Der Schlauchanschluss bzw. der Kupplungsnippel 16a bzw. 16b wird in die dafür vorgesehene Einstecköffnung (der Kupplungsmuffe 17) gesteckt. Durch Anschrauben des Griffes bzw. Handstücks 3 am Funktionsteil bzw. am Dosator 2 wird der Schlauchanschluss (dessen Kupplungsteil 15) unsichtbar und drehbar durch den Klemmring bzw. das Halteorgan 14 in einer Vertiefung bzw. Aufnahme- oder Einschrauböffnung 23 des Griffes bzw. Handstücks 3 und der Einstecköffnung der Kupplungsmuffe des Funktionsteiles bzw. Dosators 2 fixiert.

Die demontierten Einzelteile können alle gereinigt und auch autoklaviert werden. Das Funktionsteil bzw. der Dosator bleibt mit dem Dosierhebel bzw. Abzug 6 montiert und wird in einem Stück gereinigt/autoklaviert.

Bei Bedarf kann die Achse bzw. der Achsstift 7 ausgedrückt und der Hebel bzw. Abzug 6 demontiert werden.

Die Versorgungsschläuche 4 bleiben ebenfalls montiert (mit dem Kupplungsteil 15) und werden nur gereinigt, nicht aber autoklaviert.

### BEZUGSZEICHENLISTE

- 1: Applikator
- 2: Dosator
- 3: Handstück
- 4: Versorgungsschlauch
- 5: Mündung des Dosators
- 6: Abzug bzw. Dosierhebel
- 7: Achsstift
- 8: Ventilverschlusskappe
- 9: Anschlag
- 10: Anschlag
- 11: Federelement
- 12: Ventilschieber
- 12a: Führungsabschnitt des Ventilschiebers
- 12b: Brückenabschnitt des Ventilschiebers
- 12c: Dichtungsabschnitt des Ventilschiebers
- 12d: Fortsetzungsabschnitt des Ventilschiebers
- 13: Mündung
- 13a: Ventilschieberbohrung
- 14: Halteorgan
- 15: Kupplungsteil
- 16a: Kupplungsnippel zweistufig
- 16b: Kupplungsnippel einstufig
- 17: Kupplungsmuffe
- 18: Bolzengewinde Kupplungsmuffe
- 19: Mutterngewinde Handstück
- 20: Bohrung
- 21: Haltenut
- 22: Sitz
- 23: Einschrauböffnung bzw. Aufnahmeöffnung, soweit nicht zum Einschrauben ertüchtigt
- 24: erster Nippelabschnitt
- 25: zweiter Nippelabschnitt
- 26: erster Muffenabschnitt
- 27: zweiter Muffenabschnitt
- 28: Mündung
- 29: Mündung
- 30: Verbindungskanal
- 31: Verbindungskanal
- 32: Ausgabekanal
- 33: Medikamentenzerstäuber
- 34: Kupplungsrohr
- 35: Zerstäuberoberteil
- 36: Vorratsbehälter des Zerstäubers
- 37: Zerstäuberlanze
- 38: Kupplungsaufnahme
- 39: Sperrhebel
- 40: Nut
- 41: Ablage
- 42: Aufnahme
- 43: Venturidüse
- 44: Steigrohr
- 45: Druckausgleich
- 46: Schnellkupplungssystem nach Art eines Luer-Lock-Verschlusses
- 47: Innenaufnahme
- 48: Zapfen
- 49: Hohlschürze
- 50: Luer-Verbindung (einfach)
- 51: Applikationsspitze
- 52: Applikationshülse
- 53: Schild
- 54: Signal- oder Markierungsring
- 55: Politzer-Olive
- 56: Stiftaufnahmenut
- 57: (Nicht vergeben)
- 58: Stiftdurchschuböffnung
- 59: Sicherungsstift

- L: Längsachse
- M: männlicher Teil
- W: weiblicher Teil
- P: Schwenkpfeil
- H: Kompressionsrichtung und daraus resultierende Verlagerung

## Patentansprüche

1. Applikatorsystem mit einem Hals-Nasen-Ohren Applikator (1), wobei der Applikator (1) ein Anschlussstück (3) umfasst, das vorzugsweise zugleich als Handstück ausgebildet ist, sowie einen Dosator (2), einen Versorgungsschlauch (4), der den Applikator (1) mit der zu applizierenden Substanz speist, und ein mit dem Schlauch verbundenes Kupplungsteil (15), wobei das Anschlussstück (3) und der Dosator (2) lösbar, vorzugsweise werkzeuglos lösbar, miteinander gekuppelt sind, **dadurch gekennzeichnet, dass** der Dosator (2) eine Kupplungsmuffe (17) ausbildet, in die ein Kupplungsnippel (16a; 16b) des mit dem Versorgungsschlauch (4) verbundenen Kupplungsteils (15) fluiddicht eingeschoben ist oder umgekehrt, dass der Kupplungsnippel (16a; 16b) durch das Kuppeln des Dosators (2) mit dem Anschlussstück (3) in Längsachsenrichtung in der Kupplungsmuffe (17) festgesetzt wird, dass das Kupplungsteil (15) eine Haltenut (21) umfasst, wobei in betriebsbereitem Zustand ein Halteorgan (14) auf der Haltenut (21) des Kupplungsteils (15) aufgeschoben ist und das Kupplungsteil (15) in betriebsbereitem Zustand formschlüssig gegen Herausziehen aus dem Anschlussstück (3) sichert.

2. Applikatorsystem nach Anspruch 1, **dadurch gekennzeichnet, dass** die Kupplungsmuffe (17) und der Kupplungsnippel (16a; 16b) so gestaltet sind, dass sie auch im betriebsbereiten Zustand des Applikators (1) relativ zueinander rotieren können und dass das Kupplungsteil (15) so von dem Anschlussstück (3) gehalten wird, dass es auch im betriebsbereiten Zustand des Applikators (1) relativ zum Anschlussstück (3) rotieren kann.

3. Applikatorsystem nach einem der vorhergehenden Ansprüche, **dadurch gekennzeichnet, dass** das Kupplungsteil (15) in betriebsbereitem Zustand des Applikators (1) derart in dem Anschlussstück (3) eingehängt ist, dass es formschlüssig gegen Herausziehen aus dem Anschlussstück (3) in Richtung der Längsachse (L) des Schlauchs gesichert ist.

4. Applikatorsystem nach einem der vorhergehenden Ansprüche, **dadurch gekennzeichnet, dass** das Kupplungsteil (15) und das Anschlussstück (3) derart ausgestaltet sind, dass das Kupplungsstück (15) mitsamt des daran befestigt bleibenden Schlauchs werkzeuglos von dem Anschlussstück (3) getrennt werden kann, sobald der Dosator (2) von dem Anschlussstück (3) abgekuppelt ist.

5. Applikatorsystem nach Anspruch 4, **dadurch gekennzeichnet, dass** das Kupplungsteil (15) und das Anschlussstück (3) derart ausgestaltet sind, dass das Kupplungsteil (15) nach dem Abziehen eines Halteorgans (14), das das Kupplungsteil (15) in betriebsbereitem Zustand formschlüssig gegen Herausziehen aus dem Anschlussstück (3) sichert, zu der dem Dosator (2) abgewandten Seite des Anschlussstücks (3) hin aus dem Anschlussstück (3) herausgezogen werden kann.

6. Applikatorsystem nach Anspruch 5, **dadurch gekennzeichnet, dass** das Halteorgan (14) erst nach dem Abkuppeln des Dosators (2) vom Anschlussstück (3) abgezogen werden kann, vorzugweise dadurch, dass das Anschlussstück (3), das Kupplungsteil (15) und das Halteorgan (14) derart gestaltet sind, dass das Kupplungsteil (15) nach dem Abkuppeln des Dosators (2) auf der Dosatorseite des Anschlussstücks (3) so weit aus diesem herausgeschoben werden kann, dass nun das in betriebsbereitem Zustand vollständig unzugänglich innerhalb des Anschlussstücks (3) untergebrachte Halteorgan (14) zugänglich wird und abgezogen werden kann.

7. Applikatorsystem nach einem der vorhergehenden Ansprüche, **dadurch gekennzeichnet, dass** der Dosator (2) eine Kupplungsmuffe (17) aufweist, die an ihrer Außenumfangsfläche ein Außengewinde trägt, mittels dessen der Dosator (2) mit einem entsprechenden Innengewinde des Anschlussstücks (3) verschraubt werden kann, wobei das Gewinde vorzugsweise so angeordnet ist, dass es bei betriebsbereitem Applikator (1), in Richtung senkrecht zur Längsachse (L) des angrenzenden Schlauchs gesehen, zwischen dem Innengewinde des Anschlussstücks (3) und dem in die Kupplungsmuffe (17) eingeschobenen Kupplungsnippel (16a; 16b) liegt.

8. Applikatorsystem nach einem der vorhergehenden Ansprüche, **dadurch gekennzeichnet, dass** der Dosator (2) eine Kupplungsmuffe (17) aufweist, deren freie Stirnfläche bei vollständig in das Anschlussstück (3) eingeschraubtem Dosator (2) eine Anschlagfläche für das Halteorgan (14) bildet.

9. Applikatorsystem nach einem der vorhergehenden Ansprüche, **dadurch gekennzeichnet, dass** der Dosator (2) eine zweistufige Kupplungsmuffe (17) ausbildet, mit einem ersten Muffenabschnitt (26) mit einem großen Durchmesser und einem zweiten Muffenabschnitt (27) mit einem kleinen Durchmesser, wobei das Applikatorsystem unterschiedliche Kupplungsteile (15) aufweist, von denen eines einen einstufigen Kupplungsnippel (16b) ausbildet und das andere einen zweistufigen Kupplungsnippel (16a) ausbildet, und der Dosator (2) so gestaltet ist, dass nach dem Einschieben eines einstufigen Kupplungsnippels (16b) in die Kupplungsmuffe (17) Fluid in statischer Form am Dosator (2) ansteht, das nur im Zuge der Applikation fließt, und dass nach dem Einschieben eines zweistufigen Kupplungsnippels (16a) das zu applizierende Fluid dynamisch am Dosator (2) ansteht, indem es permanent durch den Dosator (2) zirkuliert, egal ob gerade appliziert wird oder nicht.

10. Applikatorsystem nach einem der vorhergehenden Ansprüche, **dadurch gekennzeichnet, dass** das Applikatorsystem einen Medikamentenzerstäuber (33) umfasst, der von dem Applikator (1) mit Fluid versorgt wird, wobei die Mündung (5) des Applikators (1) ein Kupplungsrohr (34) trägt, das fluiddicht in eine Kupplungsaufnahme (38) des Medikamentenzerstäubers (33) eingeführt werden kann, oder umgekehrt, und dort formschlüssig verrastet.

11. Applikatorsystem nach Anspruch 10, **dadurch gekennzeichnet, dass** der Medikamentenzerstäuber (33) einen Sperrhebel (39) aufweist, der formschlüssig in eine Nut (40) oder Kerbe des Kupplungsrohrs (34) einrastet.

12. Applikatorsystem nach Anspruch 11, **dadurch gekennzeichnet, dass** der Sperrhebel (39) so positioniert angeordnet ist und von dem Medikamentenzerstäuber (33) nach außen absteht, dass er mit einem Finger der den Applikator (1) an seinem Handstück (3) haltenden Hand betätigt werden kann, um die Kupplung zu lösen und den Medikamentenzerstäuber (33) abnehmen zu können.

13. Applikatorsystem nach einem der vorhergehenden Ansprüche, **dadurch gekennzeichnet, dass** am Applikatorsystem zum An- und Abkuppeln von Applikatorvorsätzen und/oder Zerstäubervorsätzen eine nach Art eines Luer-lock-Verschlusses ausgebildete Kupplung (46) vorgesehen ist.

14. Applikatorsystem nach einem der vorhergehenden Ansprüche, **dadurch gekennzeichnet, dass** zu dem Applikatorsystem mehrere unterschiedliche Signal- bzw. Markierungsringe (54) gehören, die schnellwechselbar sind, indem sie bei getrenntem Dosator (2) und Anschlussstück (3) auf eine am Dosator (2) befindliche Kupplungsmuffe (17) aufgeschoben werden und beim zum betriebsfertigen Zustand führenden Kuppeln von Dosator (2) und Handstück (3) so zwischen dem Dosator (2) und dem Handstück (3) zu liegen kommen, dass sie im Betrieb von außen erkennbar sind.

## Claims

1. Applicator system with an ear, nose and throat applicator (1), wherein the applicator (1) comprises a connector (3) which is preferably simultaneously designed as a hand piece, and a doser (2), a supply hose (4) which feeds the applicator (1) with the substance to be applied, and a coupling element (15) connected to the hose, wherein the connector (3) and the doser (2) are coupled to one another in a detachable manner, preferably in a detachable manner without tools, **characterized in that** the doser (2) forms a coupling sleeve (17) into which a coupling nipple (16a; 16b) of the coupling element (15) connected to the supply hose (4) is inserted in a fluid-tight manner, or vice versa, that, by coupling the doser (2) to the connector (3), the coupling nipple (16a; 16b) is fixed in the direction of the longitudinal axis into the coupling sleeve (17), that the coupling element (15) comprises a holding groove (21), wherein, in operational status, a holding element (14) is pushed on the holding groove (21) of the coupling element (15) and positively secures the coupling element (15) in operational status against being pulled out of the connector (3).

2. Applicator system according to claim 1, **characterized in that** the coupling sleeve (17) and the coupling nipple (16a; 16b) are designed in such a way that they can rotate relative to one another, even when the applicator (1) is ready for operation, and that the coupling element (15) is held by the connector (3) such that it can rotate relative to the connector (3), even when the applicator (1) is ready for operation.

3. Applicator system according to one of the preceding claims, **characterized in that**, when the applicator (1) is ready for operation, the coupling element (15) is mounted to the connector (3) in such a way that it is positively secured against being pulled out of the connector (3) in the direction of the longitudinal axis (L) of the hose.

4. Applicator system according to one of the preceding claims, **characterized in that** the coupling element (15) and the connector (3) are designed in such a way that the coupling element (15) together with the hose remaining attached to it can be separated from the connector (3) without tools, as soon as the doser (2) is uncoupled from the connector (3).

5. Applicator system according to claim 4, **characterized in that** the coupling element (15) and the connector (3) are designed in such a way that, after removing a holding element (14) which positively secures the coupling element (15) in the operational status against being pulled out of the connector (3), the coupling element (15) can be pulled out of the connector (3) towards the side of the connector (3) facing away from the doser (2).

6. Applicator system according to claim 5, **characterized in that** the holding element (14) can only be removed after the uncoupling of the doser (2) from the connector (3), preferably by designing the connector (3), the coupling element (15) and the holding element (14) in such a way that, after uncoupling the doser (2), the coupling element (15) can be pushed out of the connector (3) on the doser side of the connector (3) to such an extent that the holding member (14) which is located within the connector (3) and completely inaccessible during operation becomes accessible and can be removed.

7. Applicator system according to one of the preceding claims, **characterized in that** the doser (2) has a coupling sleeve (17) which has an external thread on its outer peripheral surface, by means of which the doser (2) can be screwed to a corresponding internal thread of the connector (3), wherein the thread is preferably arranged in such a way that, seen in the direction perpendicular to the longitudinal axis (L) of the adjacent hose, when the applicator (1) is ready for operation, it is positioned between the internal thread of the connector (3) and the coupling nipple (16a; 16b) inserted into the coupling sleeve (17).

8. Applicator system according to one of the preceding claims, **characterized in that** the doser (2) has a coupling sleeve (17), the free end face of which forms a stop surface for the holding member (14), when the doser (2) is completely screwed into the connector (3).

9. Applicator system according to one of the preceding claims, **characterized in that** the doser (2) forms a two-stage coupling sleeve (17), with a first sleeve section (26) having a large diameter and a second sleeve section (27) having a small diameter, wherein the applicator system comprises different coupling elements (15), one of which forms a single-stage coupling nipple (16b) and the other forms a two-stage coupling nipple (16a), and the doser (2) is designed in such a way that, after inserting a single-stage coupling nipple (16b) into the coupling sleeve (17), the fluid is present in static form at the doser (2), which fluid only flows during the application, and that, after inserting a two-stage coupling nipple (16a), the fluid to be applied is dynamically present at the doser (2) by constantly circulating through the doser (2), regardless of whether it is being applied or not.

10. Applicator system according to one of the preceding claims, **characterized in that** the applicator system comprises a medicament atomizer (33) which is supplied with fluid by the applicator, wherein the orifice (5) of the applicator (1) carries a coupling tube (34) which can be inserted in a fluid-tight manner into a coupler pocket (38) of the medicament atomizer (33), or vice versa, and engages there in a form-fitting manner.

11. Applicator system according to claim 10, **characterized in that** the medicament atomizer (33) has a locking lever (39) which engages positively into a groove (40) or notch of the coupling tube (34).

12. Applicator system according to claim 11, **characterized in that** the locking lever (39) is positioned and protrudes outward from the medicament atomizer (33) in such a way that it can be operated with a finger of the hand holding the applicator (1) on its hand piece (3) so as to loosen the coupling and to remove the medicament atomizer (33).

13. Applicator system according to one of the preceding claims, **characterized in that** a coupling (46) designed in the form of a Luer lock is provided on the applicator system for coupling and uncoupling the applicator attachments and / or atomizer attachments.

14. Applicator system according to one of the preceding claims, **characterized in that** several different signal or marking rings (54) belong to the applicator system, which rings can be replaced quickly by sliding them onto a coupling sleeve (17) located on the doser (2), when the doser (2) and the connector (3) are separated, and, during the coupling of the doser (2) and the hand piece (3), said coupling leading to the operational status, the rings lie between the doser (2) and the hand piece (3) in such a way that they can be recognized from the outside during operation.

## Revendications

1. Système applicateur avec un applicateur de nez, d'oreilles et de gorge (1), l'applicateur (1) comprenant une pièce de raccordement (3), qui est de préférence également conçue comme une pièce à main, et un doseur (2), un tuyau d'alimentation (4) qui alimente l'applicateur (1)) en la substance à appliquer, et une pièce d'accouplement (15) reliée au tuyau, la pièce de raccordement (3) et le doseur (2) étant couplés l'un à l'autre de manière détachable, de préférence de manière détachable et sans outils, **caractérisé en ce que** le doseur (2) forme un manchon d'accouplement (17) dans lequel un mamelon d'accouplement (16a; 16b) de la pièce d'accouplement (15) reliée au tuyau d'alimentation (4) est inséré de manière étanche ou, inversement, que par le couplage du doseur (2) avec la pièce de raccordement (3) le mamelon d'accouplement (16a; 16b) est fixé dans le manchon d'accouplement (17) dans le sens de l'axe longitudinal, que la pièce d'accouplement (15) comprend une rainure de fixation (21), un élément de fixation (14) étant enfilé sur la rainure de fixation (21) de la pièce d'accouplement (15) en état de fonctionnement et empêchant par la connexion mécanique la pièce d'accouplement (15) d'être retirée de la pièce de raccordement (3) en état de fonctionnement.

2. Système applicateur selon la revendication 1, **caractérisé en ce que** le manchon d'accouplement (17) et le mamelon d'accouplement (16a; 16b) sont conçus de manière à pouvoir tourner l'un par rapport à l'autre même lorsque l'applicateur (1) est prêt à fonctionner et à ce que la pièce d'accouplement (15) est tenue par la pièce de raccordement (3) de manière à pouvoir tourner par rapport à la pièce de raccordement (3), lorsque l'applicateur (1) est prêt à fonctionner.

3. Système applicateur selon l'une des revendications précédentes, **caractérisé en ce que**, lorsque l'applicateur (1) est prêt à fonctionner, la pièce d'accouplement (15) est accrochée dans la pièce de raccordement (3) de sorte que la connexion mécanique l'empêche d'être retirée de la pièce de raccordement (3) dans le sens de l'axe longitudinal (L) du tuyau.

4. Système applicateur selon l'une des revendications précédentes, **caractérisé en ce que** la pièce d'accouplement (15) et la pièce de raccordement (3) sont conçues de telle sorte que la pièce d'accouplement (15) et le tuyau restant attaché à celle-ci peuvent être séparés de la pièce de raccordement (3) sans outils dès que le doseur (2) est découplé de la pièce de raccordement (3).

5. Système applicateur selon la revendication 4, **caractérisé en ce que** la pièce d'accouplement (15) et la pièce de raccordement (3) sont conçues de telle sorte que, après avoir retiré un élément de maintien (14) qui empêche la pièce d'accouplement (15) par la connexion mécanique d'être retirée de la pièce de raccordement (3) en état de fonctionnement, la pièce d'accouplement (15) peut être retirée de la pièce de raccordement (3) vers le côté de la pièce de raccordement (3) qui est opposé au doseur (2).

6. Système applicateur selon la revendication 5, **caractérisé en ce que** l'élément de maintien (14) ne peut être retiré de la pièce de raccordement (3) qu'après le découplage du doseur (2) de la pièce de raccordement (3), de préférence **en ce que** la pièce de raccordement (3), la pièce d'accouplement (15) et l'élément de maintien (14) sont conçus de telle manière qu'après le découplage du doseur (2) la pièce d'accouplement (15) peut être retirée de la pièce de raccordement (3) du côté de la pièce de raccordement (3) faisant face au doseur (2) jusqu'à ce que l'élément de maintien (14) qui est logé à l'intérieur de la pièce de raccordement (3) et complètement inaccessible en état de fonctionnement devient alors accessible et peut être retiré.

7. Système applicateur selon l'une des revendications précédentes, **caractérisé en ce que** le doseur (2) comporte un manchon d'accouplement (17) qui a un filetage extérieur sur sa surface circonférentielle extérieure, au moyen duquel le doseur (2) peut être vissé sur un filetage interne correspondant de la pièce de raccordement (3), le filetage étant de préférence disposé de sorte que, lorsque l'applicateur (1) est prêt à fonctionner, vu dans la direction perpendiculaire à l'axe longitudinal (L) du tuyau adjacent, il se trouve entre le filetage intérieur de la pièce de raccordement (3) et le mamelon d'accouplement (16a; 16b) inséré dans le manchon d'accouplement (17).

8. Système applicateur selon l'une des revendications précédentes, **caractérisé en ce que** le doseur (2) comporte un manchon d'accouplement (17) dont la face d'extrémité libre forme une surface d'arrêt pour l'élément de maintien (14), lorsque le doseur (2) est complètement vissé dans la pièce de raccordement (3).

9. Système applicateur selon l'une des revendications précédentes, **caractérisé en ce que** le doseur (2) forme un manchon d'accouplement (17) à deux étages, avec un premier tronçon de manchon (26) ayant un grand diamètre et un deuxième tronçon de manchon (27) ayant un petit diamètre, le système applicateur comprenant différentes pièces d'accouplement (15), dont l'une forme un mamelon d'accouplement à un étage (16b) et l'autre forme un mamelon d'accouplement à deux étages (16a), et le doseur (2) est conçu de telle sorte qu'après l'insertion d'un mamelon d'accouplement à un étage (16b) dans le manchon d'accouplement (17) le fluide est présent sous forme statique au doseur (2), le fluide ne s'écoulant qu'au cours de l'application, et qu'après l'insertion d'un mamelon d'accouplement à deux étages (16a), le fluide à appliquer est présent sous forme dynamique au doseur (2) en circulant en permanence à travers le doseur (2), qu'il soit appliqué ou non.

10. Système applicateur selon l'une des revendications précédentes, **caractérisé en ce que** le système applicateur comprend un atomiseur de médicament (33) qui est alimenté par l'applicateur (1) en fluide, l'embouchure (5) de l'applicateur (1) portant un tube de couplage (34) qui peut être inséré de manière étanche dans un boitier de couplage (38) de l'atomiseur de médicament (33), ou inversement, et s'y verrouille par la connexion mécanique.

11. Système applicateur selon la revendication 10, **caractérisé en ce que** l'atomiseur de médicament (33) comprend un levier de verrouillage (39) qui s'engage par la connexion mécanique dans une rainure (40) ou encoche du tube de couplage (34).

12. Système applicateur selon la revendication 11, **caractérisé en ce que** le levier de verrouillage (39) est positionné et fait saillie de l'atomiseur de médicament (33) vers l'extérieur de manière à pouvoir être actionné d'un doigt de la main tenant l'applicateur (1) sur sa pièce à main (3)) pour desserrer l'accouplement et retirer l'atomiseur de médicament (33).

13. Système applicateur selon l'une des revendications précédentes, **caractérisé en ce qu'**un accouplement (46) conçu à la manière d'un verrouillage « Luer lock » est prévu sur le système applicateur pour coupler et désaccoupler des embouts d'applicateur et / ou des embouts d'atomiseur.

14. Système applicateur selon l'une des revendications précédentes, **caractérisé en ce que** plusieurs anneaux de signalisation ou de marquage différents (54) appartiennent au système applicateur, qui peuvent être échangés rapidement en les glissant sur un manchon d'accouplement (17) qui se trouve sur le doseur (2), lorsque le doseur(2) et la pièce de raccordement (3) sont séparés, et qui, lors du couplage du doseur (2) et de la pièce à main (3), ledit couplage conduisant à l'état de fonctionnement, se positionnent entre le doseur (2) et la pièce à main (3) de manière à pouvoir être reconnus de l'extérieur pendant le fonctionnement.
